# EUROPEAN PATENT APPLICATION

(11) **EP 4 215 204 A1**
(43) Date of publication of application: **26.07.2023**
(21) Application number: 22382050.7
(22) Date of filing: 24.01.2022
(51) Int. Cl.: A61K 38/17, A61P 17/06, A61P 17/10

(54) **HUMAN NEUTROPHIL GELATINASE-ASSOCIATED LIPOCALIN DERIVED FROM RECOMBINANT BACTERIA AND USES THEREOF**

(71) Applicant: Universitat Pompeu Fabra, 08002 Barcelona (ES)
(72) Inventor: GÜELL CARGOL, Marc, 08002 Barcelona (ES); KNÖDLSEDER, Nastassia, 08002 Barcelona (ES); FÁBREGA FERNÁNDEZ, Maria José., 08002 Barcelona (ES)
(74) Representative: Hoffmann Eitle

(57) **Abstract**

The present invention provides a human neutrophil gelatinase-associated lipocalin (NGAL) protein derived from a recombinant bacterium and its uses thereof. Particularly, the present invention relates to the use of NGAL protein obtained from a recombinant bacterium in the treatment of acne vulgaris or as a cosmetical composition. Provided herein are also recombinant bacterium expressing NGAL protein and their uses.

## Description

### TECHNICAL FIELD OF THE INVENTION

The present invention relates to the field of dermatologic disorders. In particular, the present invention relates to use of neutrophil gelatinase-associated lipocalin (NGAL) protein produced by a bacterium in the treatment of retinoid-responsive conditions, particular acne vulgaris, in a subject in need thereof.

### BACKGROUND OF THE INVENTION

Retinoids (RA) have been used as therapeutic agents for numerous skin diseases, from psoriasis to acne and wrinkles. Acne vulgaris is a common chronic skin disease involving blockage and/or inflammation of pilosebaceous units (hair follicles and their accompanying sebaceous gland). Acne can present as noninflammatory lesions, inflammatory lesions, or a mixture of both, affecting mostly the face but also the back and chest.

Acne vulgaris affects nearly 10% of people worldwide. One of the pathogenic factors for the development of acne is the excessive proliferation of *Cutibacterium acnes (C. acnes,* also called *Propionibacterium acnes* or P. *acnes),* which is the most abundant commensal of human skin and inhabits the pilosebaceous unit in the hair follicle.

Treatment include topical agents, hormonal or oral treatment. For severe acne, a systemic drug, Isotretinoin, also known as 13-cis retinoic acid (13-cis RA), is the only effective treatment. However, this drug is associated with serious side effects including depression, psychosis, anaemia and teratogenicity. Approximately 20% of acne patients develop severe acne and the great amount and severity of side effects resulting from isotretinoin treatment calls for a replacement which still can provide release of acne symptoms.

It has been shown that patients treated with Isotretinoin have a 7-fold increase in Neutrophil gelatinase- associated lipocalin 2 (NGAL) protein, and that this increase results in a decrease of sebum production, which eventually leads to a reduced count of C. *acnes.* Sebocytes treated with C. *acnes* have increased NGAL expression and secretion through a TLR-2 dependent mechanism. NGAL has thus been shown to mediate the apoptotic response of Isotretinoin in human sebocytes. Further, NGAL is not only an apoptotic agent but also has antibacterial functions, as it is able to bind to bacterial siderophores and prevents bacteria from receiving the iron necessary for cellular processes. In view of this, the most widely accepted hypothesis of the efficacy observed upon Isotretinoin treatment is that this drug causes an increase in NGAL protein which leads to both sebum reduction and antibacterial activity, reducing the proliferation of P. *acnes* in the skin of affected people.

In order to use NGAL as an alternative to Isotretinoin in the treatment of acne, the production of the recombinant protein is needed. Proteins can be produced in eukaryotic or prokaryotic cells. However, production systems based on eukaryotic cells are more expensive and less industrially scalable, while prokaryotic protein synthesis can be fairly faster and cheaper because bacteria cells are easier to produce, grow and maintain. Importantly, when a condition affects millions of people worldwide, such as acne or acne-related diseases, the scalability and cost-effectiveness of a treatment represent important factors to be considered.

Some reports show that human recombinant NGAL (rhNGAL) produced in eukaryotic cells can induce apoptosis in SEB-1 sebocytes (Nelson et al. Neutrophil gelatinase-associated lipocalin mediates 13-cis retinoic acid-induced apoptosis of human sebaceous gland cells. J Clin Invest. 2008 Apr;118(4):1468-78. doi: 10.1172/JCI33869). At the same time, other reports have shown that recombinant NGAL from prokaryotic origin (produced in *E. coli*) does not induce apoptosis in TSS-1 sebocytes (Kimberly Ruth Lumsden. The innate immune protein neutrophil gelatinase-associated lipocalin is involved in the early therapeutic response to 13-cis retinoic acid in acne patients. Doctor of Philosophy, May 2011), indicating that posttranslational modifications of NGAL intrinsic to the methods of production (such as glycosylation pattern) may have an impact in the functionality of the protein. Further, due to the antibacterial activity that NGAL has shown in prokaryotic cells, such as *E. coli, P. acnes* or *Mycobacterium tuberculosis* (Goetz, Holmes, Borregaard et al. 2002; Martineau, Newton, Wilkinson et al. 2007), strategies aimed at producing this protein using prokaryotic cells have been found not to be possible. Overall, the state of the art indicates that recombinant NGAL produced in bacteria does not have the same function or expression pattern as endogenous NGAL generated in eukaryotic cells, and thus recombinant NGAL produced in eucaryote cells, despite of being more expensive and less scalable, represents the most promising NGAL-based treatment for acne.

The present invention aims at providing an industrially scalable and cost-effective system of producing NGAL protein that is based on the use of prokaryotic cells without hampering the effectiveness of the NGAL protein in reducing sebum production.

### BRIEF DESCRIPTION OF THE INVENTION

In an aspect, the present invention relates to a human neutrophil gelatinase-associated lipocalin (NGAL) protein derived from a culture of a recombinant bacterium, for use in inhibiting sebum production in a human epithelial cell, preferably in a sebocytes, wherein said NGAL protein is obtained or obtainable from a method comprising the steps of:
a) culturing in a suitable media a recombinant bacterium characterized in that it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b).

In another aspect, the present invention relates to a human neutrophil gelatinase-associated lipocalin (NGAL) protein derived from a culture of a recombinant bacterium, for use in the treatment of acne vulgaris, urticaria, eczema, rosacea and/or psoriasis, wherein said NGAL protein is obtained or obtainable from a method comprising the steps of:
a) culturing in a suitable media a recombinant bacterium characterized in that it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b).

In another aspect, the present invention relates to a non-therapeutical cosmetic use of a human neutrophil gelatinase-associated lipocalin (NGAL) protein derived from a culture of a recombinant bacterium, wherein said NGAL protein is obtained or obtainable from a method comprising the steps of:
a) culturing in a suitable media a recombinant bacterium characterized in that it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b),
preferably to prevent, reduce and/or ameliorate skin aging.

In another aspect, the present invention relates to a method for producing the active ingredient of a pharmaceutical composition, wherein said active ingredient is a human neutrophil gelatinase-associated lipocalin (NGAL) protein that is capable of inhibiting sebum production in a human epithelial cell, preferably in a sebocyte, wherein the method comprises the steps of:
a) culturing in a suitable media a recombinant bacterium characterized in that it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b).

Preferably, the recombinant bacterium according to any of the aspects above is Cutibacterium acnes or Escherichia coli. Preferably, the human neutrophil gelatinase-associated lipocalin (NGAL) protein according to any of the aspects above has at least 85% amino acid sequence identity over the full length to SEQ ID NO: 1. More preferably for in any of the above aspects, the recombinant bacterium is Cutibacterium acnes and the human neutrophil gelatinase-associated lipocalin (NGAL) protein has at least 85% amino acid sequence identity over the full length to SEQ ID NO: 1.

In a further aspect, the present invention provides a recombinant Cutibacterium acnes characterized in that it expresses the human neutrophil gelatinase-associated lipocalin (NGAL) protein. Preferably, said human neutrophil gelatinase-associated lipocalin (NGAL) protein has at least 85% amino acid sequence identity over the full length to SEQ ID NO: Preferably, said NGAL is operably linked to a promoter selected from the list consisting of camp2 promoter, camp1 promoter or roxP promoter.

### DESCRIPTION OF THE FIGURES

**Fig. 1****.** Cytotoxicity assay of rhNGAL on human SZ95 sebocytes incubated with different concentrations from 0, 50, 100, 500, 1000 ng/mL. After 24 h cell viability was measured by MTT assay. Displayed is the average of 4 independent experiments. Cells without treatment were used as a negative control.
**Fig. 2****.** NGAL toxicity study on *C. acnes* culture. Data represents experimental duplicates treated with 0, 100, 500 or 1000 ng/mL of rhNGAL protein. The used protein was expressed and purified from E. coli BL21 cells.
**Fig. 3****.** Western blot of cytosolic (pellet) and secreted (supernatant) fraction of C. acnes pBR13, pBR14 and pBR16 compared to 40 ug (pBR13), 10 ug (pBR14) and 25 ug (pBR16) of purified protein.

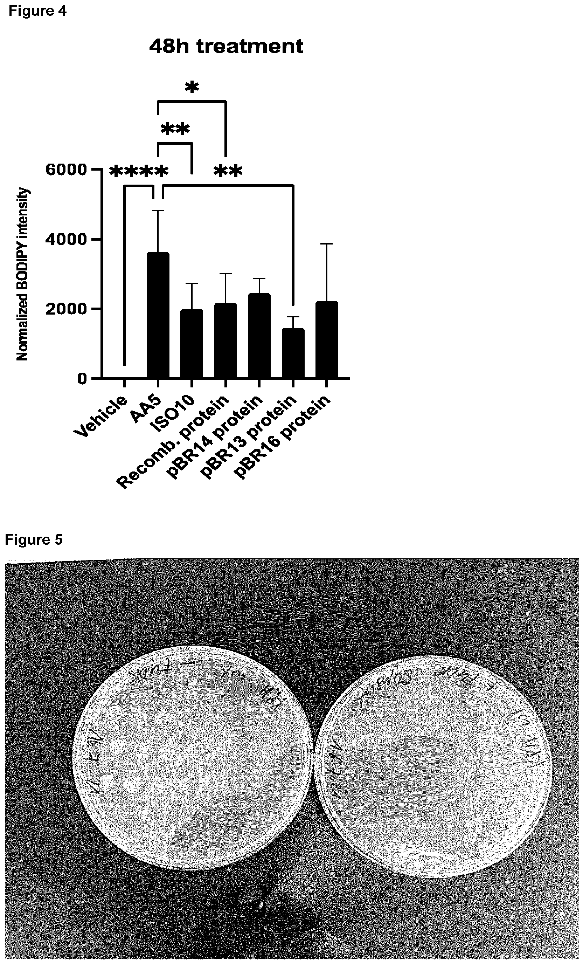

**Fig. 4****.** Sebocytes PCi-SEB Cau were treated with arachidonic acid 5 uM (AA5) to induce sebum production and treated with either 10 uM isotretinoin, rhNGAL produced in *(E. coli*) or NGAL pBR13 (produced in *C. acnes)* for 48 h. Lipids were stained by using BODIPY dye and visualized by confocal microscopy. Data is presented as normalized BODIPY intensity after treatment with NGAL from C. *acnes* pBR13 compared to Isotretinoin (10 uM) treatment and recombinant NGAL protein (50 ng/mL). Results are presented as mean standard deviation (SD) of triplicate measurements (p< 0.05) versus vehicle control.
**Fig. 5****.** TDK test (see Shao X, et al. A markerless gene deletion and integration system for Thermoanaerobacter ethanolicus. Biotechnol Biofuels. 2016;9:100. Published 2016 May 4. doi:10.1186/s13068-016-0514-1).

### DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus five percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

As used herein, the term "NGAL" has its general meaning in the art and refers to the Neutrophil Gelatinase-Associated Lipocalin as described in Schmidt-Ott K M. et al. (2007) (Schmidt-Ott K M, Mori K, Li J Y, Kalandadze A, Cohen D J, Devarajan P, Barasch J. Dual action of neutrophil gelatinase-associated lipocalin. J Am Soc Nephrol. 2007 February; 18(2):407-13. Epub 2007 Jan. 17. Review.). NGAL was shown to exist both as a 25-kDa monomer and a 45-kDa disulfide-linked homodimer, and it may also be covalently complexed with neutrophil gelatinase (also known as matrix metalloproteinase 9, MMP-9) via an intermolecular disulphide bridge as a 135-kDa heterodimeric form.

As used herein, the term "derivative" or "analogue" refers to any substance which is sufficiently structurally similar to the material of which it is identified as a derivative so as to have substantially similar functionality or activity, for example, therapeutic effectiveness, as the material when the substance is used in place of the material.

As used herein, "endogenous gene" or "endogenous promoter" refers to native nucleic acid sequences that are originating from within an organism, that is, that have not external cause. Opposite to "endogenous gene" or "endogenous promoter" is a "exogenous gene" or "exogenous promoter" or "heterologous promoter" or "heterologous gene", which refer to nucleotide sequences that have an external origin, i.e., that are not native or naturally found in said organism. An example of an exogenous gene, in the context of the present invention, is the nucleic acid encoding for the NGAL protein.

As used herein, the term "retinoid-responsive conditions" refers to a number of pathological conditions that are currently treated using retinoids or to those pathological conditions for which administration of one or more retinoids has a beneficial effect, including those characterized by an excessive production of sebum by the skin, such as *acne vulgaris.* Other retinoid-responsive conditions rosacea, urticaria, eczema and psoriasis. Retinoids are well-known in the art and include retinol, retinal, tretinoin, isotretinoin, alitretinoin, etretinate, acitretin, tazarotene, bexarotene and adapalene.

As used herein, the expression "non-therapeutic use in the cosmetic field" refers to a method used to improve a person's appearance, i.e., to beautify appearance. The used in cosmetics does not involve a treatment by surgery or therapy.

The term "isolated" as used herein refers to a substance that has been separated from contaminating cellular components associated with the substance in nature not intended to be associated with the substance and that would interfere with use of the substance in therapeutic, prophylactic, diagnostic or other uses. Generally, an isolated substance described herein is at least about 80% pure, at least about 90% pure, at least about 95% pure, or greater than about 99% pure. Purification is achieved using well-known standard methodology such as fractionation and/or chromatography.

The term "recombinant" refers to protein or bacteria that are not wildtype, that is, that is not found in nature are thus formed by laboratory methods. By "recombinant bacterium" is referred to a bacterium altered, modified or engineered (e.g., genetically engineered) such that it exhibits an altered, modified or different genotype or phenotype (e.g., when the genetic modification affects coding nucleic acid sequences of the microorganism) as compared to the naturally- occurring or starting microorganism from which it was derived.

The terms "treating" and "treatment" used to refer to treatment of a retinoid-responsive condition in a subject includes: preventing, inhibiting or ameliorating the retinoid-responsive condition in a subject, such as slowing progression of the condition and/or reducing or ameliorating a sign or symptom of the condition.

The term "therapeutically effective amount" refers to an amount which produces a desired physiologic or pharmacologic effect in a subject, prevents or ameliorates a condition being treated in the subject. For example, a therapeutically effective amount is an amount which reduces or eliminates a sign or symptom of a retinoid-responsive condition being treated in the subject.

The term "subject" as used herein is a living multi-cellular vertebrate organisms, including, for example, humans, non-human mammals and (non-human) primates.

### DESCRIPTION

As stated above, there is scientific evidence supporting the use of NGAL as an alternative to Isotretinoin in the treatment of acne. However, up to date, the production of NGAL protein can only be based on eukaryotic systems, as NGAL produced in bacteria did not induce apoptosis in the TSS-1 sebocyte model. One possible reason that NGAL produced by bacteria does not function as expected may be because of the specific glycosylation pattern provided by eukaryotic cells. Klausen et al. showed that recombinant NGAL produced in *E. coli* failed to induce apoptosis in human myeloid bone marrow cells (Klausen, Niemann, Cowland et al. 2005), and NGAL has been reported to be an N-glycosylated protein when expressed in eukaryotic cells. However, prokaryotic cells do not glycosylate proteins in the same manner as mammalian cells, and it may be that the glycosylation of NGAL in eukaryotic cells aides in the recognition of the protein by its receptor.

Bacteria have long been the favourite expression system for recombinant protein production. However, in the case of NGAL production, it is also known that NGAL has antibacterial activity, which means that its production in a bacterial-based system would probably not be workable.

The authors of the present invention have surprisingly found that, although it is reported that NGAL has antibacterial activity against C. *acnes,* recombinant bacteria (such as P. *acnes or E. coli*) can indeed be grown and used to produce NGAL. Further, and most importantly, the NGAL produced by C. *acnes* and *E. coli* and secreted to the supernatant of the bacterial culture was found effective in reducing sebum in Sebocytes PCi-SEB Cau (see Fig. 4). These results provide evidence that functional NGAL can be recombinantly produced in a bacterial system and that it maintains its inhibitory effect, opening the path for the production of NGAL at an industrially scalable levels and the use of this protein to treat acne worldwide.

In view of this, a **first** aspect of the present invention relates to a neutrophil gelatinase-associated lipocalin (NGAL) protein derived from the culture of a bacterium, for use in therapy or as a medicament. In an embodiment of the first aspect, the NGAL protein derived from the culture of a bacterium is used in inhibiting sebum production in an epithelial cell. In a preferred embodiment, the neutrophil gelatinase-associated lipocalin (NGAL) protein derived from the culture of a bacterium, is used in the treatment of diseases characterized by an excess of sebum production, preferably *acne vulgaris.* By *"acne vulgaris"* is referred herein as the skin condition that occurs when the hair follicles become plugged with sebum and dead skin cells, usually leading the overgrow of bacteria. *Acne vulgaris* includes the presence of whiteheads, blackheads or pimples, or a combination thereof. More particularly, *acne vulgaris* include the presence of small red, tender bumps, papules, closed plugged pores, open plugged pores, pustules, nodules, and cystic lesions, or any combination thereof. The *acne vulgaris* condition may be present in the face, forehead, chest, upper back, and shoulders, or in a combination thereof, of the subject to be treated. The *acne vulgaris* may be severe or mild *acne vulgaris.*

Retinoids, a huge family of compounds derived from vitamin A, are known to regulate epithelial cell growth, where they inhibit differentiation of sebaceous glands. As shown in the Examples below, it was found that NGAL produced by recombinant bacteria is able to inhibit sebum production in sebocytes, rendering more than plausible that NGAL according to the first aspect may also be useful in the treatment of other retinoid-responsive diseases, such as urticaria, eczema, dermatitis, rosacea and/or psoriasis.

Preferably, NGAL derived from a culture of a recombinant bacterium is used in inhibiting sebum production in an epithelial cell. Preferably, the epithelial cell is a human skin cell, more preferably a human sebocyte. Sebocytes are highly specialized, sebum-producing epithelial cells that release their content by rupture of the cell membrane and cellular degradation. Sebocytes are terminally differentiated epithelial cells that produce and accumulate lipids (sebum). These cells are most commonly found in the skin in association with hair follicles (forming the pilosebaceous unit), where they arise from hair follicle keratinocytes, but there are also sebaceous glands (SGs) not associated with a hair follicle. Sebum synthesis by sebocytes is strongly regulated by hormones, in particular by androgens. Excessive sebum production is seen in *acne vulgaris,* one of the most common skin diseases. Histologically, sebocytes can be commonly identified by lipophilic dyes such as Oil Red O, Nile Red and Sudan IV, or by immunostaining against lipid droplet associated proteins such as perilipin and adipophilin, the enzyme fatty acid synthase, keratin 7 and other specific differentiation markers. Methods to measure the sebum inhibiting effect of NGAL provided herein are known in the art and are also illustrated in the examples below. In an embodiment, the NGAL derived from the culture of a recombinant bacterium is capable of cause a reduction in sebum production in a NGAL-treated cell, preferably in a sebocyte, wherein said reduction is in about 1.1, 1.2, 1.3, 1.4, 1.5, 1.6, 1.7, 1.8, 1.9, 2, 2.1, 2.2, 2.3, 2.4, 2.5, 3, 4, 6, 8, 10-fold or more than 10-fold reduction as compared to the sebum production in an untreated cell. In an embodiment, the reduction in sebum production caused by the NGAL provided herein is a statistically significant reduction as compared to sebum production of an untreated cell, preferably an untreated sebocyte.

In a second aspect, the present invention provides non-therapeutic indications or uses of a neutrophil gelatinase-associated lipocalin (NGAL) protein derived from the culture of a bacterium in the cosmetic field. Preferably, the NGAL protein obtained or obtainable from the culture of a bacterium is used with the non-therapeutic purposes of cleaning, beautifying, adding to the attractiveness, altering the appearance, or keeping or promoting the skin or hair in good condition. Said purposes may also be whitening, minimizing the appearance of lines in the face and body, protecting from the sun and sun tanning. In an embodiment, the non-therapeutic use is to prevent, reduce and/or ameliorate skin aging.

In some embodiments, the bacterium according to the first or the second aspect is from the genus *Cutibacterium* or *Escherichia.* More preferably, the bacterium is *Cutibacterium acnes* (C. *acnes)* or *Escherichia coli.* Please note that *Cutibacterium acnes* was formerly known as *Propionibacterium acnes (P. acnes),* and thus, for the purpose of the present invention, both names are considered synonymous and interchangeable. *Cutibacterium acnes* is a gram-positive, anaerobic bacteria that normally occupies the hair follicles and sebaceous glands. The *Cutibacterium acnes* to be used in the present invention can be any, as long as it enables the introduction of a nucleic acid encoding for the NGAL protein and its expression. Preferably, the C. *acnes* is KPA171202 strain. *Escherichia coli,* on the other hand, also known as *E. coli,* is a Gram-negative, facultative anaerobic, rod-shaped, coliform bacterium of the genus Escherichia that is commonly found in the environment, foods, and intestines of people and animals. The *E. coli* to be used in the present invention to produce the NGAL protein can be any, as long as it enables the introduction of a nucleic acid encoding for the NGAL protein and its expression. Preferably, the *E. coli* is BL21 strain.

The recombinant bacterium, more preferably C. *acnes* or *E. coli,* is characterized in that it comprises a nucleic acid molecule encoding for the NGAL protein. Thus, the recombinant bacterium described herein is thus able to express the NGAL protein. In an embodiment, the nucleic acid molecule encoding for the NGAL protein comprises or consists of a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 2. Preferably, the NGAL protein expressed by the recombinant bacterium, preferably recombinant C. *acnes* or *E. coli,* according to the present invention is encoded by a nucleic acid that consists of SEQ ID NO: 2. In an embodiment, the nucleic acid that encodes for the NGAL protein is codon optimized, preferably codon optimized for the bacterium in which it will be produced, such as codon optimized for *E. coli* or C. *acnes.*

In an embodiment, the NGAL protein derived from the culture of a bacterium, for use according to the first or second aspects is from human origin. Preferably, the NGAL protein expressed by the recombinant bacterium, preferably recombinant C. *acnes or E. coli,* comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length with SEQ ID NO: 1. Preferably, the NGAL protein expressed by the recombinant bacterium, preferably recombinant C. *acnes* or *E. coli,* consists of SEQ ID NO: 1.

Techniques for determining sequence identity between nucleic acids and amino acids are known in the art. Two or more sequences can be compared by determining their "percent identity." The percent identity of two sequences, whether nucleic acid or amino acid sequences, is the number of exact matches between two aligned sequences divided by the length of the shorter sequences and multiplied by 100. "Percent (%) amino acid sequence identity" with respect to proteins, polypeptides, antigenic protein fragments, antigens and epitopes described herein is defined as the percentage of amino acid residues in a candidate sequence that are identical with the amino acid residues in the reference sequence (i.e., the protein, polypeptide, antigenic protein fragment, antigen or epitope from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST, ALIGN, or Megalign (DNASTAR) software. Those skilled in the art can determine appropriate parameters for measuring alignment, including any algorithms needed to achieve maximum alignment over the full-length of the sequences being compared. The same applies to "percent (%) nucleotide sequence identity", mutatis mutandis.

Genes encoding NGAL protein are preferably designed to either facilitate secretion of the protein from the bacterium or to facilitate expression of the NGAL protein directly on the surface of the bacterium. In some embodiments, the NGAL protein produced by the bacterium defined herein is released to the extracellular media when the bacterium is growing and thus is metabolic active. Thus, in an embodiment, the NGAL protein derived from the culture of a recombinant bacterium, for use according to the first or the second aspect is obtained from the supernatant media collected from the culture of said recombinant bacterium, preferably the recombinant C. *acnes* or *E. coli.* Preferably, the NGAL protein is obtained from the supernatant collected after a period of growth under suitable conditions. Said period may be 6, 8, 10, 12, 14, 16, 18, 20, 22, 24, 48, 72 hours or more. For the protein to be recovered from the supernatant, it should comprise in its amino acid sequence a signal peptide that leads its secretion to the extracellular medium. A signal peptide is a small amino acid sequence whose has function is to direct the protein to the outside of the cytoplasm. Some signal peptides such as PeIB, TorA, OmpA represent secretion pathway that is commonly used for extracellular expression in bacteria. Preferably, the signal peptide is placed at the N-terminal of the NGAL protein. In a preferred embodiment, the signal peptide has an amino acid sequence which comprises or consists of SEQ ID NO: 6, 7, or 8, or an amino acid sequence with at least 70%, 75%, 80%, 85%, 90%, 91%, 92%, 93%, 94%, 95%, 96%, 97%, 98%, 99%, or 100% identity over the full length of SEQ ID NO: 6, 7 or 8.

Thus, in a preferred embodiment, the NGAL protein obtained or obtainable from the culture of a bacterium, preferably recombinant C. *acnes* or *E. coli,* for use according to the first or the second aspects is secreted to the extracellular media, and it comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 9, 10 or 11. Preferably, the NGAL protein expressed by the recombinant bacterium, preferably recombinant C. *acnes* or *E. coli,* consists of SEQ ID NO: 9, 10 or 11.

In addition, for working the present invention, any recombinant NGAL protein such as derivatives or analogues can be used, as long as they are obtained from bacteria. "Derivatives" or "analogues" of NGAL include truncated version of the protein where the function of the NGAL is not altered by said truncation. "Derivatives" or "analogues" also include fusion proteins that are longer than the wild-type NGAL protein due to the addition of extra sequences, such as signal peptides, leader peptides, etc.

In order to obtain a recombinant bacterium that expresses the NGAL protein as defined herein, a nucleotide sequence encoding for said protein should be introduced in a bacterium to provide a recombinant bacterium expressing NGAL. Different means and methods to introduce exogenous nucleotide sequences and thus produce recombinant bacteria are known in the art, such as conventional transformation methods, electroporation, conjugation or protoplast transformation.

The introduction of DNA encoding a heterologous antigen into a bacterium, to produce a recombinant bacterium may be accomplished, for example, by the creation of a recombinant bacterium in which DNA encoding the NGAL protein is harboured on a vector, such as a plasmid. Alternatively, DNA encoding the NGAL protein may be stably integrated into the bacterium chromosome by employing, for example, transposon mutagenesis or by homologous recombination. A preferred method for producing recombinant bacteria comprising a nucleotide encoding for the NGAL protein integrated into the chromosome thereof, is the induction of homologous recombination between a plasmid transfer vector comprising DNA encoding the NGAL protein and bacterium chromosomal DNA. In some embodiments, the nucleotide sequence encoding the NGAL protein, preferably human NGAL protein, is located in a plasmid transfer vector that is transformed into the bacterium, preferably C. *acnes or* E. *coli.* Integration of the nucleic acid encoding the human NGAL protein is performed preferentially by homologous recombination. Homologous recombination events are exchanges between DNA molecules (in this case, between bacterial genome and the plasmid transfer vector) that share enough sequence identity to trigger a homologous recombination mechanism between them. Techniques to promote homologous recombination are known in the art, and they lead to the introduction of a desired portion of the plasmid transfer vector into the bacterial genome. To produce by homologous recombination the recombinant bacterium expressing NGAL, preferably the C. *acnes* or *E.coli,* a plasmid transfer vector comprising the NGAL-encoding nucleic acid flanked by two regions (one upstream and another downstream of the NGAL coding sequence) of at least 50 base pairs that are homologous to a region in the genome of the bacterium needs to be construed. Once said plasmid transfer vector is provided, it is transformed into the bacterium, and the homologous recombination event between the bacterial genome and the plasmid transfer vector will result in the recombinant bacterium comprising NGAL gene in its genome and thus expressing the NGAL protein. Methods to produce a recombinant bacterium are known in the art, see, e.g., Sorensen et al. Mutagenesis of Propionibacterium acnes and analysis of two CAMP factor knock-out mutants, Journal of Microbiological Methods,Volume 83, Issue 2,2010,Pages 211-216,ISSN 0167-7012,https://doi.org/10.1016/j.mimet.2010.09.008. Examples of a transfer vector to lead the homologous recombination is provided in the Example section below.

In a preferred embodiment, the nucleic acid encoding the NGAL protein is incorporated into a non-essential region of the genome of the bacterium. Preferably, the nucleic acid sequence encoding for the human NGAL protein is inserted into a gene selected from the list consisting of camp2 (PPA0687), camp1 (PPA1340), roxP (PPA1939), thymidine kinase (tdk) (PPA1049), or Restriction methylation IIIB locus (PPA1610, 1611, 1612). Said genes encode for the camp2, camp1, RoxP, Tdk, Restriction methylation IIIB proteins, respectively, and thus the insertion of NGAL encoding nucleic acid leads to the interruption or even the replacement of said genes. As shown in Fig. 5, the interruption or replacement of tdk locus represents a promising tool for a knock-out C. *acnes expressing* NGAL that is able to grow in Brucella plates with FUDR as a negative selection.

For the homologous recombination event to occur specifically in said genes of the bacterial genome, the plasmid transfer vector used should comprise flanking regions that present homology to said genes, wherein said flanking regions are located upstream and downstream of the NGAL-encoding nucleic acid. Thus, in a preferred embodiment, the plasmid transfer vector that will guide the insertion of the nucleic acid sequence encoding the NGAL protein into the bacterial genome comprises a region comprising, in the following order:
i) a nucleotide region of at least 50, 100 or 150 base pairs, preferably 200, 250, 300, 350, 400, 452, 500, or more than 500 base pairs, wherein said nucleotide region is at least 70%, 80%, 85%, 90%, 95%, or 100% identical to a nucleotide region of the same length comprised in the bacterial genes camp2 (PPA0687), camp1 (PPA1340), roxP (PPA1939), tdk (PPA1049), or Restriction methylation IIIB locus (PPA1610, 1611, 1612),
ii) a NGAL nucleic acid encoding for the human NGAL protein, preferably the NGAL nucleic acid of SEQ ID NO: 2, or a nucleic acid with at least 85%, 90%, 91%, 92%, 93%, 94%, 95%, 96%, 97%, 98%, 99%, or 100% identity over the full length to SEQ ID NO: 2, and
iii) a nucleotide region of least 50, 100 or 150, preferably 200, 250, 300, 350, 400, 452, 500, or more than 500 base pairs, wherein said nucleotide region is at least 70%, 80%, 85%, 90%, 95%, or 100% identical to a nucleotide region of the same length comprised in the bacterial genes camp2 (PPA0687), camp1 (PPA1340) roxP (PPA1939), tdk (PPA1049), or Restriction methylation IIIB locus (PPA1610, 1611, 1612,
so that the NGAL nucleic acid is inserted by homologous recombination into the genome of the bacterium, interrupting the genes camp2 (PPA0687), camp1 (PPA1340) roxP (PPA1939), tdk (PPA1049), or Restriction methylation IIIB locus (PPA1610, 1611, 1612) from the bacterial genome, preferably C. *acnes* or *E. coli* genomes. An example of said vector for targeting the tdk locus is defined in SEQ ID NO: 15 and in the Examples section below.

Other methods known in the field of gene engineering useful for generating recombinant bacteria can be used, such as, CRISPR, phage integration, or transposon insertion. In one embodiment, the nucleic acid encoding for the NGAL protein is comprised in a plasmid that is transformed into the bacterium. In another embodiment, the nucleic acid construct is a shuttle plasmid. In another embodiment, the nucleic acid construct is an integration vector comprising an integration site. In another embodiment, the nucleic acid construct is a site-specific integration vector. In another embodiment, the nucleic acid construct is any other type of nucleic acid construct known in the art.

It is noted that the nucleotide sequence introduced in the recombinant bacterium may comprise not only the gene encoding for the NGAL protein, but also other elements such as regulatory sequences or selectable markers. In one embodiment, a regulatory sequence is a promoter, while in another embodiment, a regulatory sequence is an enhancer.

In some embodiments, the nucleic acid comprised in the recombinant bacterium comprises an expression cassette comprising the nucleic acid encoding for the NGAL protein, operably linked to a promoter. The promoter driving the expression of NGAL protein may be an endogenous promoter, i.e., a promoter that is naturally found in the bacterium where the NGAL protein is being expressed, preferably C. *acnes* or *E. coli.* In this case, it is not necessary to include the promoter in the plasmid transfer vector, as it is already present in the bacterial genome. In some embodiments, the insertion of the nucleic acid encoding for the NGAL protein into the camp2 (PPA0687), camp1 (PPA1340), roxP (PPA1939), tdk (PPA1049), or Restriction methylation IIIB locus (PPA1610, 1611, 1612) genes results in said nucleic acid being operably linked to the endogenous camp2, camp1, roxP, Tdk, or Restriction methylation IIIB promoters, respectively, so that said endogenous promoters control the expression of the NGAL protein.

In some other embodiments, the promoter is an artificial promoter or an exogenous promoter. In this case, the promoter needs to be supplied by, e.g., the transfer plasmid vector, together with the NGAL encoding gene. In some other embodiments, the camp2, camp1, roxP, Tdk, or Restriction methylation IIIB promoters, or any other suitable promoter, is also included in the transfer vector and is flanked by the regions that lead homologous recombination, so that the NGAL nucleotide sequence is not operably linked to an endogenous promoter, but its expression will be driven by the promoter included in the transfer plasmid vector, preferably by camp2, camp1, roxP, Tdk, or Restriction methylation IIIB promoters, and which is also inserted into the bacterial genome when the homologous recombination event takes place.

In an embodiment, the NGAL protein obtained or obtainable from the culture of a bacterium, and for use according to the first or second aspects of the present invention, is encoded by a nucleic acid which is operably linked to a promoter selected from the list of camp2, camp1, roxP, Tdk, or Restriction methylation IIIB promoters. Preferably the NGAL protein obtained or obtainable from the culture of a bacterium, and for use according to the first or second aspects of the present invention, is encoded by a nucleic acid that is operably linked to a promoter comprising or consisting of a nucleotide sequence with at least 50%, 60%, 70%, 75%, 80%, 85%, 90%, 91%, 92%, 93%, 94%, 95%, 96%, 97%, 98%, 99%, or 100% identity over the full length with SEQ ID NO: 12 (camp1 promoter), 13 (RoxP promoter), or 14 (camp2 promoter).

As described in the examples, several nucleotides were generated comprising NGAL nucleic acid operably linked to one of the promoters camp2, camp1,or roxP, wherein the NGAL proteins produced further comprised a signal peptide (named PPA1939, PPA0687, PPA1340) at their N-terminal (see table 1). Thus, in an embodiment, the NGAL protein obtained or obtainable from the culture of a bacterium, and for use according to the first or second aspects of the present invention, comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 1, 9, 10, or 11 and said NGAL protein is encoded by a nucleic acid that is operably linked to a promoter comprising or consisting of a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 12 (camp1 promoter), 13 (RoxP promoter), or 14 (camp2 promoter). Preferably, the NGAL protein obtained or obtainable from the culture of a bacterium, and for use according to the first or second aspects of the present invention, comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 10 and said NGAL protein is encoded by a nucleic acid that is operably linked to a promoter comprising or consisting a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 12. Preferably, the NGAL protein obtained or obtainable from the culture of a bacterium, and for use according to the first or second aspects of the present invention, comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 11 and said NGAL protein is encoded by a nucleic acid that is operably linked to a promoter comprising or consisting a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 12. Most preferably, the NGAL protein obtained or obtainable from the culture of a bacterium, and for use according to the first or second aspects of the present invention, comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 9 and said NGAL protein is encoded by a nucleic acid that is operably linked to a promoter comprising or consisting a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 12.

As stated above, the nucleic acid transformed in the bacterium may comprise the nucleic acid sequence encoding for a selectable marker. The selectable marker may impart resistance to one or more antibiotic agents. For example, selectable markers may include genes for ampicillin resistance, streptomycin resistance, kanamycin resistance, tetracycline resistance, chloramphenicol resistance, sulphonamide resistance, erythromicine resistance, lincomycin resistance or combinations of these markers. Typically, the selectable marker is operationally linked to a promoter that facilitates expression of the marker. Plasmids and other cloning vectors that include selectable markers are known in the art.

Once the step of introducing a nucleic acid encoding for a NGAL protein into a bacterium to obtain a recombinant bacterium expressing NGAL protein, as defined above, is performed, the NGAL protein used according to the first and second aspects is obtained or obtainable from a method comprising the steps of:
a) culturing a recombinant bacterium in a suitable media and under suitable conditions for protein expression, wherein said recombinant bacterium is characterized in that it expresses a human neutrophil gelatinase-associated lipocalin (NGAL) protein, preferably a secretory NGAL protein as defined above,
b) collecting the NGAL protein from the culture of a), preferably from the supernatant of the culture of a),
c) optionally, isolating the NGAL collected in b).

Suitable culture media include any growth medium in a solid, liquid, or semisolid state that allow the growth of the recombinant bacterium and the expression of the NGAL protein. Suitable culture media thus comprise all the elements needed for the bacterium to grow and to be metabolic active, including at least one carbon source, water, salts, and at least one source of amino acid and nitrogen. Several approaches may be employed to express the heterologous antigen in bacteria as will be understood by one skilled in the art once armed with the present disclosure.

The produced NGAL protein may be obtained from the interior of the bacterium, by for instance cell lysis and optionally purification or it may be obtained from the extracellular media if the NGAL protein has been designed to be a secretory protein. In the latter case, the NGAL protein is obtained from the extracellular media directly or after centrifuging said media to discard bacterial cells. Optionally, said NGAL is purified before being used. Protein purification comprises a series of processes intended to isolate one or a few proteins from a complex mixture, such as the culture media where the producer recombinant bacterium is growing. The purification process separates the NGAL protein from all other proteins and compounds present in the media. Separation steps usually exploit differences in protein size, physicochemical properties, binding affinity and biological activity, which are known by the skilled person. Optionally, the purified protein is further concentrated prior use. Stable transformants of which express NGAL may be isolated and characterized as described herein in the experimental examples.

The NGAL protein derived from the culture of a bacterium, for use according to the first or second aspects of the present invention may be administered to the subject in a single dose, or in multiple (i.e., 2, 3, 4, etc.) doses. A suitable schedule for administration of doses depends on several factors including age, weight, gender, medical history and health status of the subject, type of composition used and route of administration. One of skill in the art is able to readily determine a dose and schedule of administration for a particular subject. Suitable dosages ranges depend on various factors such as the age of the subject, the severity and type of condition being treated in the subject, the general condition of the subject, the route and form of administration of the composition being administered and the particular composition administered. In the case of the therapeutic use according to the first aspect, one of ordinary skill in the art will be able to ascertain a therapeutically effective amount without undue experimentation in view of the art. In some embodiments, the administration may be at the onset of the symptoms of the acne vulgaris condition, for instance, once the first hair follicles become plugged.

In certain embodiments, the subject in need thereof is a human. In certain embodiments, the subject is a teenager, preferably between the ages of 10 and 19 years. In other embodiments, the subject to be treated or in need thereof is an adult. In some embodiments, the subject has acne.

In a particular embodiment of the present invention a method of treating and/or preventing acne in a subject is provided, which includes administering a therapeutically effective amount of NGAL, an NGAL analogue, a supernatant comprising NGAL, or a C. *acnes* or *E. coli* expressing NGAL, as defined above.

The subject treated according to the uses of the present invention can be mammalian or non-mammalian. Humans are preferred subjects, although and a mammalian subject can be any mammal including, but not limited to, a non-human primate; a rodent such as a mouse, rat, or guinea pig; a domesticated pet such as a cat or dog; a horse, cow, pig, sheep, goat, or rabbit.

Since the human NGAL protein derived from the recombinant bacterium according to the first or second aspects may be secreted to the extracellular media, in a **third** aspect, the present invention relates to the supernatant media obtained or obtainable from a culture of a recombinant bacterium, preferably C. *acnes or E. coli,* wherein the bacterium is characterized by expressing the NGAL protein, wherein said NGAL protein comprises a signal peptide that leads its excretion to the extracellular medium. In some embodiments, the supernatant further comprises bacteria in suspension, preferably recombinant C. *acnes or E. coli.* In some embodiments, the supernatant has been treated, e.g. by centrifugation or filtration to remove any remaining bacteria. Optionally, the NGAL obtained from the supernatant is purified before being used. The uses defined in the first and second aspect also apply for the supernatant of the third aspect. Thus, the therapeutic use and the non-therapeutic cosmetic use defined above are also uses of the supernatant according to the third aspect.

It is important to note that, although the NGAL protein (purified or comprised in the supernatant) can be used as defined in the above aspects, it is of great interest to also provide a recombinant vector that is able to efficiently produce and deliver said protein preferably in its niche of action, i.e., in the hair follicle, which is surrounded by sebocytes. As shown in Paetzhold et al 2019, C. *acnes* can engraft and stay on human skin, being a promiser producer and shuttle for the therapeutic NGAL protein directly on human skin. Thus, in a **fourth aspect,** the present invention relates to a recombinant bacterium, preferably C. *acnes* or *E. coli,* characterized in that it comprises a NGAL encoding nucleic acid and characterized in that said recombinant bacterium expresses a neutrophil gelatinase-associated lipocalin (NGAL) protein, preferably a human NGAL. Preferably, the recombinant bacterium, preferably C. *acnes* or *E. coli,* is characterized in that it expresses a NGAL protein that comprises or consists of an amino acid sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 1, 9, 10, or 11. Preferably, the NGAL protein expressed by the recombinant bacterium, preferably recombinant C. *acnes* or *E. coli,* consists of SEQ ID NOs: 1, 9, 10, or 11. Preferably, the recombinant bacterium, preferably C. *acnes* or *E. coli,* is characterized in that it expresses an NGAL protein that is encoded by a nucleic acid that comprises or consists of a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 2. Preferably, the recombinant bacterium has been transformed with a nucleic acid molecule that comprises a nucleic acid encoding for the NGAL protein operably linked to a transcription promoter, preferably camp1, camp2 or roxP promoters. Optionally, said NGAL protein is encoded by a nucleic acid sequence that is operably linked and under the control of a promoter comprising or consisting a nucleotide sequence with at least 50%, 60%, 70%, 75%, 80%, 85%, 90%, 91%, 92%, 93%, 94%, 95%, 96%, 97%, 98%, 99%, or 100% identity over the full length to SEQ ID NO: 12 (camp1 promoter), 13 (RoxP promoter), or 14 (camp2 promoter).

Embodiments regarding the recombinant bacteria, preferably recombinant C. *acnes* or *E. coli,* and methods to produce said recombinant bacteria, are explained above and also apply here. The embodiments related to the NGAL protein and its sequence as described in the previous aspects also apply here.

Further, in a **fifth** aspect, the present invention provides a recombinant bacterium as defined in the fourth aspect, preferably C. *acnes* or *E. coli,* characterized in that it expresses a NGAL protein, preferably a human NGAL, said NGAL as defined in any of the previous aspects, for use in therapy or as a medicament. Preferably, the recombinant bacterium is used in inhibiting sebum production in a human skin cell or in the treatment of diseases characterized by an excess of sebum production, such as *acne vulgaris.* Other uses of the recombinant bacterium according to the fifth aspect include the treatment of other retinoid-responsive diseases, such as urticaria, eczema, dermatitis, rosacea and/or psoriasis. Preferably, the recombinant bacterium for use in therapy or as a medicament is C. *acnes,* as it provides the benefit of targeted delivery and increased protein penetration since this bacteria is a natural commensal in the hair follicle, being thus in direct contact to human cells like sebocytes.

In a sixth aspect, the present invention provides a recombinant bacterium, preferably C. *acnes* or *E. coli,* characterized in that it expresses a NGAL, preferably a human NGAL, as defined in any of the previous aspects, for the non-therapeutic use in the cosmetic field or as a cosmetic composition. In a preferred embodiment, the recombinant bacterium for the non-therapeutic cosmetic use is C. *acnes* expressing NGAL, preferably human NGAL. Preferably, the recombinant bacterium, preferably C. *acnes* or *E. coli,* characterized in that it expresses a NGAL protein, is used with the non-therapeutic purposes of cleaning, beautifying, adding to the attractiveness, altering the appearance, or keeping or promoting the skin or hair in good condition. Said purposes may also be whitening, minimizing the appearance of lines in the face and body, protecting from the sun and sun tanning. In an embodiment, the non-therapeutic cosmetic use is to prevent, reduce and/or ameliorate skin aging.

In a seventh aspect, the present invention relates to a composition, preferably a pharmaceutical composition or a cosmetic composition, comprising the human NGAL derived from the culture of the recombinant bacterium as defined above, preferably by C. *acnes or E. coli,* or a supernatant obtained from the culture of said bacterium, and a pharmaceutically or cosmetically acceptable carriers. Since C. *acnes* is a common commensal of skin microbiota and can thus be administered, at least topically, to a subject, also included in the seventh aspect of the present invention is a composition, preferably a pharmaceutical or cosmetic composition, comprising the recombinant C. *acnes* according to any of the previous aspects characterized in that it expresses NGAL protein, wherein the pharmaceutical or cosmetical composition further comprises a pharmaceutically or cosmetically acceptable carrier. Also included in the seventh aspect of the present invention is a composition, preferably a pharmaceutical or cosmetic composition, comprising the recombinant *E. coli* according to any of the previous aspects characterized in that it expresses NGAL protein, wherein the pharmaceutical or cosmetical composition further comprises a pharmaceutically or cosmetically acceptable carrier.

In some embodiments, the composition comprises a preparation of substantially pure, preferably of up to 80%, 85%, 90%, 95% or 100% pure, isolated NGAL protein derived from the culture of a recombinant bacterium expressing NGAL. In some other embodiments, the composition, preferably a pharmaceutical composition or a cosmetic composition, comprises a preparation of substantially pure, preferably of up to 80%, 85%, 90%, 95% or 100% pure, recombinant C. *acnes* or *E. coli* expressing NGAL protein.

Preferably, the pharmaceutical composition comprises a therapeutically effective amount of NGAL protein, preferably isolated NGAL protein, or a therapeutically effective amount of C. *acnes,* or a combination thereof.

The term "pharmaceutically acceptable carrier" refers to a carrier which is suitable for use in a subject without undue toxicity or irritation to the subject and which is compatible with other ingredients included in a pharmaceutical composition. Pharmaceutically acceptable carriers, methods for making pharmaceutical compositions and various dosage forms, as well as modes of administration are well-known in the art. Other substances that may be comprised in the composition include approved additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like.

In some embodiments, the pharmaceutical composition is formulated for topical, local and/or systemic administration to the subject. Formulations to administer the pharmaceutical composition according to the seventh aspect include those suitable for oral, rectal, nasal, pulmonary, ocular, otic, intraarterial, intradermal, intravenous, intramuscular, intraosseous, subcutaneous, topical, transdermal, and transmucosal, such as by sublingual, buccal, vaginal, and inhalational routes of administration.

The pharmaceutical or cosmetic composition according to embodiments of the present invention is in any dosage form suitable for administration to a subject, illustratively including solid, semi-solid and liquid dosage forms such as tablets, capsules, powders, granules, suppositories, pills, solutions, suspensions, ointments, lotions, creams, gels, pastes, sprays and aerosols. For topical administration, a composition can be formulated for administration to the skin such as for local effect, and/or as a "patch" formulation for transdermal delivery. Pharmaceutical formulations suitable for topical administration include, for example, ointments, lotions, creams, gels, pastes, sprays and powders. Ointments, lotions, creams, gels and pastes can include, in addition to one or more active agents, a base such as an absorption base, water-removable base, water-soluble base or oleaginous base and excipients such as a thickening agent, a gelling agent, a colorant, a stabilizer, an emulsifying agent, a suspending agent, a sweetener, a flavouring, or a perfuming agent. Powders and sprays for topical administration of one or more active agents can include excipients such as talc, lactose and one or more silicic acids. Sprays can include a pharmaceutical propellant such as a fluorinated hydrocarbon propellant, carbon dioxide, or a suitable gas. Alternatively, a spray can be delivered from a pump-style spray device which does not require a propellant. A spray device delivers a metered dose of a composition contained therein, for example, using a valve for regulation of a delivered amount. Transdermal formulations can include percutaneous absorption enhancers such as acetone, azone, dimethyl acetamide, dimethyl formamide, dimethyl sulfoxide, ethanol, oleic acid, polyethylene glycol, propylene glycol and sodium lauryl sulfate. lonotophoresis and/or sonophoresis can be used to enhance transdermal delivery.

Liposomes and emulsions are well-known types of pharmaceutical formulations that can be used to deliver a pharmaceutical agent, particularly a hydrophobic pharmaceutical agent. Liposomes can include any type of amphipathic materials compatible with a composition to be delivered, illustratively including naturally-occurring lipids, synthetic lipids and combinations thereof.

The term "cosmetically acceptable carrier" refers to a carrier which is suitable for the non-therapeutic use in a subject without undue toxicity or irritation. The cosmetically acceptable carrier may comprise or consist of a cream, gel, serum, balm, sun cream, after sun cream, foundation, tinted cream, tinted sun cream, soothing anti redness cream with green tint, scalp serum, solution, suspension, emulsion, ointment, foam, paste, lotion, powder, soap, surfactant-containing cleansing oil or spray. Dosage forms, as well as modes of administration are well-known in the art. The step of applying the cosmetic composition to the skin may be repeated once or twice daily for up to 8 days. The step of applying the cosmetic composition to the area of skin may be repeated for at least two days, at least three days, at least four days, at least six days, at least seven days, at least 10 days, at least two weeks, at least three weeks, at least four weeks, at least six weeks or at least 8 weeks.

In some embodiments, the composition may be provided in kits for one or more than one administration.

In an eighth aspect, the present invention relates to a method for producing the active ingredient of a pharmaceutical composition, wherein said active ingredient is a NGAL protein that is capable of inhibiting sebum production in an epithelial cell, preferably a sebocyte, wherein the method comprises the steps of:
a) introducing a nucleic acid encoding for a NGAL protein into a bacterium to obtain a recombinant bacteria expressing NGAL protein, preferably a secretory NGAL protein,
b) culturing in a suitable media said recombinant bacterium,
b) collecting the NGAL protein from the culture of a), preferably from the supernatant of the culture of a),
c) optionally, isolating the NGAL collected in b).

Since the eighth aspect refers to a NGAL protein and a bacterium producing said protein, all the embodiments disclosed above relating to NGAL protein and recombinant bacteria expressing said NGAL protein should be considered included in the eighth aspect.

### SEQUENCE LISTING

**NGAL amino acid sequence without signalling peptide (SEQ ID NO: 1):** 
QDSTSDLIPAPPLSKVPLQQNFQDNQFQGKWYVVGLAGNAILREDKDPQKMYATIYELKED
KSYNVTSVLFRKKKCDYWIRTFVPGCQPGEFTLGNIKSYPGLTSYLVRVVSTNYNQHAMVF
FKKVSQNREYFKITLYGRTKELTSELKENFIRFSKSLGLPENHIVFPVPIDQCIDG*
**NGAL nucleotide sequence without signalling peptide (SEQ ID NO: 2):** 
CAGGACTCCACCTCCGACCTCATCCCGGCCCCGCCGCTCTCCAAGGTCCCGCTCCAGC
AGAACTTCCAGGACAACCAGTTCCAGGGCAAGTGGTACGTCGTCGGCCTCGCCGGCAA
CGCCATCCTCCGCGAGGACAAGGACCCGCAGAAGATGTACGCCACCATCTACGAGCTC
AAGGAGGACAAGTCCTACAACGTCACCTCCGTCCTCTTCCGCAAGAAGAAGTGCGACTA
CTGGATCCGCACCTTCGTCCCGGGCTGCCAGCCGGGCGAGTTCACCCTCGGCAACATC
AAGTCCTACCCGGGCCTCACCTCCTACCTCGTCCGCGTCGTCTCCACCAACTACAACCA
GCACGCCATGGTCTTCTTCAAGAAGGTCTCCCAGAACCGCGAGTACTTCAAGATCACCC
TCTACGGCCGCACCAAGGAGCTCACCTCCGAGCTCAAGGAGAACTTCATCCGCTTCTC
 
CAAGTCCCTCGGCCTCCCGGAGAACCACATCGTCTTCCCGGTCCCGATCGACCAGTGC
ATCGACGGC*
**PPA1939 secretion peptide nucleotide sequence: (roxP) (SEQ ID NO: 3):** atgttcgtccaaatcgctgccagcctggcagccgcatcgtccattgcactcggcataccaggagctgcc
**PPA0687 secretion peptide nucleotide sequence: (Camp2) (SEQ ID NO: 4):**
   atgaagaagacccatcttgtagctcccctccttgtcggcgcaatgctcgtaccagcggcgctgtcagctcccagtgctcatgct
**PPA1340 secretion peptide nucleotide sequence: (camp1) (SEQ ID NO: 5):**
   atgaaggttaagttcttagcagcgccgctggttgttggtgccttgatggcgccggcagctttctctggagcgacagctcatgct
**PPA1939 secretion peptide amino acid sequence: (roxP) (SEQ ID NO: 6):**
   MFVQIAASLAAASSIALGIPGAA
**PPA0687 secretion peptide amino acid sequence: (Camp2) (SEQ ID NO: 7):**
   MKKTHLVAPLLVGAMLVPAALSAPSAHA
**PPA1340 secretion peptide amino acid sequence: (camp1) (SEQ ID NO: 8):**
   MKVKFLAAPLVVGALMAPAAFSGATAHA
NGAL amino acid sequence with signal peptide **PPA1939 (SEQ ID NO: 9):** 
MFVQIAASLAAASSIALGIPGAAQDSTSDLIPAPPLSKVPLQQNFQDNQFQGKWYVVGLAG
NAILREDKDPQKMYATIYELKEDKSYNVTSVLFRKKKCDYWIRTFVPGCQPGEFTLGNIKSY
PGLTSYLVRVVSTNYNQHAMVFFKKVSQNREYFKITLYGRTKELTSELKENFIRFSKSLGLPE
NHIVFPVPIDQCIDG*
NGAL amino acid sequence with signal peptide **PPA0687 (SEQ ID NO: 10):** 
MKKTHLVAPLLVGAMLVPAALSAPSAHAQDSTSDLIPAPPLSKVPLQQNFQDNQFQGKWY
VVGLAGNAILREDKDPQKMYATIYELKEDKSYNVTSVLFRKKKCDYWIRTFVPGCQPGEFTL
GNIKSYPGLTSYLVRVVSTNYNQHAMVFFKKVSQNREYFKITLYGRTKELTSELKENFIRFSK
SLGLPENHIVFPVPIDQCIDG*
NGAL amino acid sequence with signal peptide **PPA1340 (SEQ ID NO: 11):** 
MKVKFLAAPLVVGALMAPAAFSGATAHAQDSTSDLIPAPPLSKVPLQQNFQDNQFQGKW
YVVGLAGNAILREDKDPQKMYATIYELKEDKSYNVTSVLFRKKKCDYWIRTFVPGCQPGEFT
LGNIKSYPGLTSYLVRVVSTNYNQHAMVFFKKVSQNREYFKITLYGRTKELTSELKENFIRFS
KSLGLPENHIVFPVPIDQCIDG*
**PPA1340 promoter sequence: (Camp1) (SEQ ID NO: 12):** 
TAGGTTGACTGGAAAGGGGATATGACACCCATCGTCCTGGGTCTGACAGGGGTAGACA
ATATAGCTTAGAAATGTGTATAAGTAAGATGTTTCTCGCAATGAAAGACGATATGTCACG
AGTTTTCTGGTGCTGTTTCCCGACACGGAACCGTTAGTTTGAATCAATTTTGAACTGAGG
ACACCAAGTTGACCCCCTGGTGTGCAACGATGCTTCTTGCTTGCAGTTGCGAGCAATTG
TTCCCGATGAAAGGAACCCACAA
**PPA1939 promoter sequence: (RoxP) (SEQ ID NO: 13):** 
ACACCGACGATCTGCACGGGCGAGTCTAGGGGTTATCGGCTACATGCGCGTCGCCGCA
TCAGGTTCACGAAACCGAAAGGCAGACCCACCCCCTACCGGGCATACCGTTGCCGCCG
ACACTTCGATGACACTATGCAGTGCTACACTTCAACTGAAAATCTATATGATTTTCGTCA
ATTCCCATACGTACGACACATCGGGAGATCGTC
**PPA0687 promoter sequence: (Camp2): (SEQ ID NO: 14):** 
gcaacaagcctagacgggtctatgcaccgtcgtagccatacaccacacgggcccctgtcggaatgacgcacgcctgatcacag
ctccaacaaatgttggcggcgtacacgccgttcaccacaccgacccccatccaaaatatccgatacacttcgcttgcctcgaccg
gggcatccgcaaccaataaacggagaaccttt
**pBR13 full plasmid sequence: suicide vector for targeting tdk locus (5139 bp): (SEQ ID NO: 15):**
   **UPPERCASE BOLD:** PPA1340 promoter sequence: (Camp1) (SEQ ID NO: 12)
   **lowercase bold:** roxP secretion peptide (SEQ ID NO: 3)
   underlined: LCN2 gene encoding for NGAL (SEQ ID NO: 2) 
ttgccgggaagctagagtaagtagttcgccagttaatagtttgcgcaacgttgttgccattgctacaggcatcgtggtgtcacgctcgt
cgtttggtatggcttcattcagctccggttcccaacgatcaaggcgagttacatgatcccccatgttgtgcaaaaaagcggttagctc
cttcggtcctccgatcgttgtcagaagtaagttggccgcagtgttatcactcatggttatggcagcactgcataattctcttactgtcatg
ccatccgtaagatgcttttctgtgactggtgagtactcaaccaagtcattctgagaatagtgtatgcggcgaccgagttgctcttgccc
ggcgtcaatacgggataataccgcgccacatagcagaactttaaaagtgctcatcattggaaaacgttcttcggggcgaaaactc
tcaaggatcttaccgctgttgagatccagttcgatgtaacccactcgtgcacccaactgatcttcagcatcttttactttcaccagcgttt
ctgggtgagcaaaaacaggaaggcaaaatgccgcaaaaaagggaataagggcgacacggaaatgttgaatactcatactctt
cctttttcaatattattgaagcatttatcagggttattgtctcatgagcggatacatatttgaatgtatttagaaaaataaacaaataggg
gttccgcgcacatttccccgaaaagtgccacctgatgcggtgtgaaataccgcacagatgcgtaaggagaaaataccgcatca
ggaaattgtaagcgttaatattttgttaaaattcgcgttaaatttttgttaaatcagctcattttttaaccaataggccgaaatcggcaaa
atcccttataaatcaaaagaatagaccgagatagggttgagtgttgttccagtttggaacaagagtccactattaaagaacgtgga
 
ctccaacgtcaaagggcgaaaaaccgtctatcagggcgatggcccactacgtgaaccatcaccctaatcaagttttttggggtcg
aggtgccgtaaagcactaaatcggaaccctaaagggagcccccgatttagagcttgacggggaaagccggcgaacgtggcg
agaaaggaagggaagaaagcgaaaggagcgggcgctagggcgctggcaagtgtagcggtcacgctgcgcgtaaccacca
cacccgccgcgcttaatgcgccgctacagggcgcgtccattcgccattcaggctgcgcaactgttgggaagggcgatcggtgcg
ggcctcttcgctattacgccagctggcgaaagggggatgtgctgcaaggcgattaagttgggtaacgccagggttttcccagtcac
gacgttgtaaaacgacggccagtgaattgtaatacgactcactatagggcgaattgggcccgacgtcgcatgctcccggccgcc
atggcggccgcgggAATTCGCCCTTccggccgccatggcgaaggatggcagtcggcagcatggagatcgcgcctcat
gtgggttgacgagctcgttggtgctcttctcgatgagctcgatcctggaaccgccctggtcgtcactgctgatcacggcatggttgac
atccccgacgaccaccgcgtcatggtggaggacgagggtggactgctcgctgacgttgccttgttggctggtgagcctcggttccg
ccacatttacactcgtcgtcccgatgaggtcgcgcaacgatggcgacgcgtactcggggatcgtgctcaggttgtcattggcgacg
atgctgttgcggctgacctgtttggaccagttgacgtgtctacccgttcccgtttcggtgacgtactagcgattactcgtggtgattggg
ccatcatgagccgcgctacccccaaggaactcaccctcgtgggcatgcacggatcgctcactccgtgtgaaatgcggatcccatt
gctggcccagatctgtgaaggatgagggGTACCTAGGTTGACTGGAAAGGGGATATGACACCCATC
GTCCTGGGTCTGACAGGGGTAGACAATATAGCTTAGAAATGTGTATAAGTAAGATGTT
TCTCGCAATGAAAGACGATATGTCACGAGTTTTCTGGTGCTGTTTCCCGACACGGAAC
CGTTAGTTTGAATCAATTTTGAACTGAGGACACCAAGTTGACCCCCTGGTGTGCAACG
ATGCTTCTTGCTTGCAGTTGCGAGCAATTGTTCCCGATGAAAGGAACCCACAAATGGC
TAGCatgttcgtccaaatcgctgccagcctggcagccgcatcgtccattgcactcggcataccaggagctgccCAG
GACTCCACCTCCGACCTCATCCCGGCCCCGCCGCTCTCCAAGGTCCCGCTCCAGCAGA
ACTTCCAGGACAACCAGTTCCAGGGCAAGTGGTACGTCGTCGGCCTCGCCGGCAACGC
CATCCTCCGCGAGGACAAGGACCCGCAGAAGATGTACGCCACCATCTACGAGCTCAAG
GAGGACAAGTCCTACAACGTCACCTCCGTCCTCTTCCGCAAGAAGAAGTGCGACTACTG
GATCCGCACCTTCGTCCCGGGCTGCCAGCCGGGCGAGTTCACCCTCGGCAACATCAAG
TCCTACCCGGGCCTCACCTCCTACCTCGTCCGCGTCGTCTCCACCAACTACAACCAGCA
CGCCATGGTCTTCTTCAAGAAGGTCTCCCAGAACCGCGAGTACTTCAAGATCACCCTCT
ACGGCCGCACCAAGGAGCTCACCTCCGAGCTCAAGGAGAACTTCATCCGCTTCTCCAA
GTCCCTCGGCCTCCCGGAGAACCACATCGTCTTCCCGGTCCCGATCGACCAGTGCATC
GACGGCTCTAGAATCGAAGGTCGTGGCAAGCCGATCCCGAACCCGCTCCTCGGCCTCG
ACTCCACCCGCACCGGCCACCACCATCACCACCATTAGTCGAGCCCGGAGGAGAAGAG
ACTGTGAACACGAACATGGGCCCACCCCTCAAGAGGAATGGGCCCATGCCGTAAATGA
TTTCGATGCAAGTGTCAGGACAACAGCAGGgtaccatggctccaggtcctgacgagcgtggccccagggtt
cgcgtgcggtcaaggattttcccgcggtgggtggtatatcaggtatgcggggtggggccgccgaacatgatctcatcccaagaag
ggatcgacgcccgcttcgatttacgcggtttgcgcttcttgcgggggcggctaccctcctcgacgagggcgtcctgctggggagtct
catcggcagcttgagctgacgcttgtgagcgagaagtcgaagtcgaggtcttcttcgtcgatgtgcgtagcttcgacgccgcagtat
cctgcgcggaggtgtcgtcatggggtggctgggcctcgcttttccccccagcctgatctggttgggatgtctcgatggcatgcggag
ccccggctggctggggagcctggggcgtagctggctcaggatcctgattcacctccggatgagacgcagcgtcaaggactggg
gtggcgagaccttcatagatgttgaccgagtcctcttgaaacccggcaagcacactagtgaattAAGGGCGaattcgcggcc
 
gcctgcaggtcgaccatatgggagagctcccaacgcgttggatgcatagcttgagtattctatagtgtcacctaaatagcttggcgt
aatcatggtcatagctgtttcctgtgtgaaattgttatccgctcacaattccacacaacatacgagccggaagcataaagtgtaaag
cctggggtgcctaatgagtgagctaactcacattaattgcgttgcgctcactgcccgctttccagtcgggaaacctgtcgtgccagct
gcattaatgaatcggccaacgcgcggggagaggcggtttgcgtattgggcgctcttccgcttcctcgctcactgactcgctgcgctc
ggtcgttcggctgcggcgagcggtatcagctcactcaaaggcggtaatacggttatccacagaatcaggggataacgcaggaa
agaacatgtgagcaaaaggccagcaaaaggccaggaaccgtaaaaaggccgcgttgctggcgtttttccataggctccgccc
ccctgacgagcatcacaaaaatcgacgctcaagtcagaggtggcgaaacccgacaggactataaagataccaggcgtttccc
cctggaagctccctcgtgcgctctcctgttccgaccctgccgcttaccggatacctgtccgcctttctcccttcgggaagcgtggcgct
ttctcatagctcacgctgtaggtatctcagttcggtgtaggtcgttcgctccaagctgggctgtgtgcacgaaccccccgttcagccc
gaccgctgcgccttatccggtaactatcgtcttgagtccaacccggtaagacacgacttatcgccactggcagcagccactggta
acaggattagcagagcgaggtatgtaggcggtgctacagagttcttgaagtggtggcctaactacggctacactagaagaacag
tatttggtatctgcgctctgctgaagccagttaccttcggaaaaagagttggtagctcttgatccggcaaacaaaccaccgctggta
gcggtggtttttttgtttgcaagcagcagattacgcgcagaaaaaaaggatctcaagaagatcctttgatcttttctacggggtctgac
gctcagtggaacgaaaactcacgttaagggattttggtcatgagattatcaaaaaggatcttcacctagatccttttaaattaaaaat
gaagttttaaatcaatctaaagtatatatgagtaaacttggtctgacagttaccaatgcttaatcagtgaggcacctatctcagcgatc
tgtctatttcgttcatccatagttgcctgactccccgtcgtgtagataactacgatacgggagggcttaccatctggccccagtgctgc
aatgataccgcgagacccacgctcaccggctccagatttatcagcaataaaccagccagccggaagggccgagcgcagaag
tggtcctgcaactttatccgcctccatccagtctattaattg
**pBR13 full plasmid sequence: replicative vector derivative of pBRESP36A (9215 bp): (SEQ ID NO: 16)**
**UPPERCASE BOLD:** PPA1340 promoter sequence: (Camp1) (SEQ ID NO: 12)
   **lowercase bold:** roxP secretion peptide (SEQ ID NO: 3)
   underlined: LCN2 gene encoding for NGAL (SEQ ID NO: 2)

GAATTCAAGCTTGTCGACGTTAACCTGCAGGCATGCGGATCCGGTACCAGCCCGACCC
GAGCACGCGCCGGCACGCCTGGTCGATGTCGGACCGGAGTTCGAGGTACGCGGCTTG
CAGGTCCAGGAAGGGGACGTCCATGCGAGTGTCCGTTCGAGTGGCGGCTTGCGCCCG
ATGCTAGTCGCGGTTGATCGGCGATCGCAGGTGCACGCGGTCGATCTTGACGGCTGGC
GAGAGGTGCGGGGAGGATCTGACCGACGCGGTCCACACGTGGCACCGCGATGCTGTT
GTGGGCTGGACAATCGTGCCGGTTGGTAGGATCCAGCGGTGAGCAGTTCGGACGAGC
AGCCGCGCCCGCGTCGCCGCAACCAGGATCGGCAGCACCCCAACCAGAACCGGCCGG
TGCTGGGCCGTACCGAGCGGGACCGCAACCGGCGCCAGTTCGGGCAGAACTTCCTCC
GCGACCGCAAGACCATCGCGCGCATCGCCGAGACAGCCGAGCTGCGGCCCGATCTGC
CGGTGCTGGAAGCCGGCCCCGTCGAAGGGCTGCTCACCAGGGAACTCGCCGACCGCG
CGCGTCAGGTGACGTCGTACGAGATCGACCCCCGGCTGGCGAAGTCGTTGCGGGAGA

AGCTTTCCGGCCACCCGAACATCGAAGTCGTCAACGCCGACTTCCTCACCGCCGAACC
GCCGCCCGAGCCGTTCGCCTTCGTCGGCGCGATCCCCTACGGCATCACCTCGGCGAT
CGTGGACTGGTGCCTGGAGGCGCCGACGATCGAGACGGCGACGATGGTCACGCAGCT
GGAGTTCGCCCGGAAGCGGACCGGCGATTACGGCCGCTGGAGCCGCCTCACGGTGAT
GACCTGGCCGCTGTTCGAGTGGGAGTTCGTCGAGAAGGTCGACCGCCGGCTGTTCAA
GCCGGTGCCCAAGGTCGACTCGGCGATCATGCGGCTGCGCAGGCGCGCCGAACCGCT
GCTGGAAGGCGCGGCGCTCGAACGCTACGAGTCGATGGTCGAGCTGTGCTTCACCGG
CGTCGGCGGCAACATCCAGGCGTCGCTTCTGCGCAAGTACCCGAGGCGCCGCGTCGA
GGCGGCGTTCGACCACGCGGGGGTCGGGGGCGGCGCCGTGGTCGCCTACGTCCGGC
CGGAGCAGTGGCTCCGGCTGTTCGAGCGGCTGGATCAGAAGAACGAACCGAGGGGTG
GGCAGCCCCAGCGGGGCAGGCGAACCGGCGGACGGGACCACGGGGACCGGCGAACC
GGCGGGCAGGATCGCGGCGATCGGCGAACCGGCGGCCGCGACCACAGGGACCGGCA
AGCCAGCGGCCACGGCGATCGTCGCAGCAGCGGACGCAATCGCGACGACGGACGAAC
CGGCGAGCGCGAGCAGGGGGACCAAGGCGGGCGGCGGGGGCCGTCCGGGGGTGGA
CGGACCGGCGGACGTCCAGGGCGACGCGGCGGACCCGGGCAGCGGTAGTCCCCGGC
ACGCGGAACGCGGCAGGCCGTCGAGCGGCCTGCCCCGTTCTGTCGAGAGGAATCAGA
GGTTGATGTCGGCCCGGAGGTCGATGTCGCGCGACGACGAGCCGATCTCCACCGCTC
GCTTGCCGCCCCCGAGCTTCCAGCCGCCCGCGGCTTCGTCCCAGTGCTGGAGGGCCG
CTCCGCGACGTGCACCCGGACGCGCTTGGTCTCGCCCGGTGCGAGTTCGACCTTCTG
GTACCTAGGTTGACTGGAAAGGGGATATGACACCCATCGTCCTGGGTCTGACAGGGG
TAGACAATATAGCTTAGAAATGTGTATAAGTAAGATGTTTCTCGCAATGAAAGACGAT
ATGTCACGAGTTTTCTGGTGCTGTTTCCCGACACGGAACCGTTAGTTTGAATCAATTTT
GAACTGAGGACACCAAGTTGACCCCCTGGTGTGCAACGATGCTTCTTGCTTGCAGTTG
CGAGCAATTGTTCCCGATGAAAGGAACCCACAAATGGCTAGCatgttcgtccaaatcgctgcca
gcctggcagccgcatcgtccattgcactcggcataccaggagctgccCAGGACTCCACCTCCGACCTCAT
CCCGGCCCCGCCGCTCTCCAAGGTCCCGCTCCAGCAGAACTTCCAGGACAACCAGTTC
CAGGGCAAGTGGTACGTCGTCGGCCTCGCCGGCAACGCCATCCTCCGCGAGGACAAG
GACCCGCAGAAGATGTACGCCACCATCTACGAGCTCAAGGAGGACAAGTCCTACAACG
TCACCTCCGTCCTCTTCCGCAAGAAGAAGTGCGACTACTGGATCCGCACCTTCGTCCCG
GGCTGCCAGCCGGGCGAGTTCACCCTCGGCAACATCAAGTCCTACCCGGGCCTCACCT
CCTACCTCGTCCGCGTCGTCTCCACCAACTACAACCAGCACGCCATGGTCTTCTTCAAG
AAGGTCTCCCAGAACCGCGAGTACTTCAAGATCACCCTCTACGGCCGCACCAAGGAGC
TCACCTCCGAGCTCAAGGAGAACTTCATCCGCTTCTCCAAGTCCCTCGGCCTCCCGGA
GAACCACATCGTCTTCCCGGTCCCGATCGACCAGTGCATCGACGGCTCTAGAATCGAA
GGTCGTGGCAAGCCGATCCCGAACCCGCTCCTCGGCCTCGACTCCACCCGCACCGGC
CACCACCATCACCACCATTAGTCGAGCCCGGAGGAGAAGAGACTGTGAACACGAACAT
GGGCCCACCCCTCAAGAGGAATGGGCCCATGCCGTAAATGATTTCGATGCAAGTGTCA

GGACAACAGCAGGATCATCCACGCAATGAAAGCCCGCACCCAATACCTCGACCAGCTC
TAACCAAGAAAGGAACCTGATGAGCGACCAGCTAGACAGCGACCGCAACTACGACCCG
ATGATCTTCGACGTGATGCGCGAGACCGCGAACCGCGTCGTCGCCACGTACGTTGCAT
GGGAAGATGAAGCCGCTGATCCCCGCGAGGCTGCGCACTGGCAGGCCGAGCGATTCC
GCACCCGGCACGAGGTGCGCGCCGTCGACCCTGACAGCCGGCGAGCAGTTCAGGCGA
AGATCGCACAGCTGCGCGAGGAACTAGCCGCAATGCCCGAACACGCCCCAGCCATCC
CTTGGAGCAGGTGGCAGCGTCAGGGGAGTCGGGGGATGTTTGGCAGGGGATGTGGAA
AGAGAGTTCGCTTTGCTCACATGGCTCAACCGGGTAACTAACTGATATGGGGTCTTCGT
CGCCCACTTTGAACACGCCGAGGAATGGACCACGCTGAACGTGACTCGCATGCTTCAC
TGCATGTATGGATTCGTTCGAGACGTTGTTCCCTGAGAGCTGGCTGCCACGCAAGCCG
CTGGCGTCAGCCGAGAAGTCTGGGGCGTACCGGCACGTGACTCGGCAGAGGGCGCTG
GAGCTGCCTTACATCGAAGCGAACCCGTTGGTCATGCAGTCCTTGGTCATCACCGATCG
AGATGCTTCGGATGCTGACTGGGCCGCAGACCTCGCTGGGCTGCCTTCACCGTCCTAC
GTGTCCATGAACCGTGTCACGACCACCGGACACATCGTCTATGCCTTGAAGAACCCTGT
GTGTCTGACCGATGCCGCGCGGCGACGGCCTATCAACCTGCTCGCCCGCGTCGAGCA
GGGCCTATGCGACGTTCTCGGCGGCGATGCATCCTACGGGCACCGGATCACAAAGAAC
CCGCTCAGCACCGCCCATGCGACCCTCTGGGGCCCCGCAGACGCGCTCTACGAGCTG
CGCGCCCTCGCACACACCCTCGACGAGATCCACGCACTGCCGGAGGCAGGGAACCCG
CGTCGCAACGTCACCCGATCAACGGTCGGCCGCAACGTCACCCTGTTCGACACCACCC
GCATGTGGGCATACCGGGCCGTCCGGCACTCCTGGGGCGGCCCGGTCGCCGAATGG
GAGCACACCGTATTCGAGCACATCCACCTACTGAACGAGACGATCATCGCCGACGAATT
CGCCACAGGCCCCCTCGGCTTGAACGAACTTAAGCACTTATCTCGATCCATTTCCCGAT
GGGTCTGGCGCAACTTCACCCCCGAAACCTTCCGCGCACGCCAGAAAGCGATCAGCCT
CCGTGGAGCATCCAAAGGCGGCAAAGAAGGCGGCCACAAAGGCGGCATTGCCAGTGG
CGCATCACGGCGCGCCCATACCCGTCAACAGTTCTTGGAGGGTCTCTCATGACCACAC
GTGAACGTCTCCCCCGCAACGGCTACAGCATCGCCGCTGCTGCGAAAAAGCTCGGTGT
CTCCGAGTCCACCGTCAAGCGGTGGACTTCCGAGCCACGCGAGGAGTTCGTGGCCCG
CGTTGCCGCACGCCACGCGCGGATTCGTGAGCTCCGCTCGGAGGGTCAGAGCATGCG
TGCGATTGCTGCCGAGGTCGGGGTTTCCGTGGGCACCGTGCACTACGCGCTGAACAAG
AATCGAACTGACGCATGACCGTAACGCCGCACGATGAGCATTTTCTTGATCGTGCACCG
CTTGGCACTACGTTCGCGTGCGGTTGCACAGTGCGCGCCACGTTCTTATCCTGCGGCC
ATTGTGGCTACAGCCAATGGGGGGCATCAGCAACGGACGTTGAACCCGGTGGGCAAGT
GTTACTCAGGGGGACATGCCCAGTCTGCGGCGCTCGGATTGACGGTATGGCAGTCGTG
CATGCGGCCCCACCGTCAAACTCATTCAGGTATCAGTGAGAACCCTCATGGCACCCCCT
CGTGACACGTTCTCGTTGCGATCAGCTGCGGCCAAGCTTGATCTTCGGGCAGCGTTGG
GTCCTGGCCACGGGTGCGCATGATCGTGCTCCTGTCGTTGAGGACCCGGCTAGGCTG
GCGGGGTTGCCTTACTGGTTAGCAGAATGAATCACCGATACGCGAGCGAACGTGAAGC

GACTGCTGCTGCAAAACGTCTGCGACCTGAGCAACAACATGAATGGTCTTCGGTTTCCG
TGTTTCGTAAAGTCTGGAAACGCGGAAGTCAGCGCCCTGCACCATTATGTTCCGGATCT
GCATCGCAGGATGCTGCTGGCTACCCTGTGGAACACCTACATCTGTATTAACGAAGCGC
TGGCATTGACCCTGAGTGATTTTTCTCTGGTCCCGCCGCATCCATACCGCCAGTTGTTT
ACCCTCACAACGTTCCAGTAACCGGGCATGTTCATCATCAGTAACCCGTATCGTGAGCA
TCCTCTCTCGTTTCATCGGTATCATTACCCCCATGAACAGAAATCCCCCTTACACGGAG
GCATCAGTGACCAAACAGGAAAAAACCGCCCTTAACATGGCCCGCTTTATCAGAAGCCA
GACATTAACGCTTCTGGAGAAACTCAACGAGCTGGACGCGGATGAACAGGCAGACATC
TGTGAATCGCTTCACGACCACGCTGATGAGCTTTACCGCAGCTGCCTCGCGCGTTTCG
GTGATGACGGTGAAAACCTCTGACACATGCAGCTCCCGGAGACGGTCACAGCTTGTCT
GTAAGCGGATGCCGGGAGCAGACAAGCCCGTCAGGGCGCGTCAGCGGGTGTTGGCG
GGTGTCGGGGCGCAGCCATGACCCAGTCACGTAGCGATAGCGGAGTGTATACTGGCTT
AACTATGCGGCATCAGAGCAGATTGTACTGAGAGTGCACCATATGACCCGAACATGTCG
CGTGGGTGCAAAATGAGCTCAACGAAACACCCCGACAAATCCTCGGCGGCGCAACCCC
ACGTGAAATACTTAACGAACTATTCAAGCGTGGCGCATCCACCGCTTGATTCCGCCTTG
AAGGCATGTTAGAGTTTGGGGCATGTCGAGTCCCGAATCAGACAGGTCAGGGGTTGTC
GGTCACACCGCCAGCCCCTTGAACCACTAGTTACGACGCCCACGATCTGTGTGGGCGT
ATGTCTGGCGTACCCGGGGCGCTGGCCGTGGTGACAAGAAGAACCATTTCTTGATCTC
ACACCTCGGAGTACTCGATGCCTTTTGCCCTCTACATGCTTGCCCTGGCGGTCTTCGTC
ATGGGCACTTCAGAATTCATGCTCGCGGGATTGCTCCCCGCGATCGCGACCGAACTTG
ACGTCTCGGTCGGCACTGCGGGCCTGCTGACCTCCGCATTCGCAGTCGGTATGGTCGT
CGGCGCGCCAGTGATGGCGGCATTCGCTCGCCGTTGGCCACCGCGGCTCACATTGAT
CGTTTGCCTTCTCGTGTTCGCGGGAAGCCACGTCATCGGAGCGATGACACCAGTGTTC
TCTCTCCTGCTCATCACCCGGGTGCTCAGCGCTCTCGCAAACGCAGGATTCCTCGCCG
TAGCACTGAGCACGGCCACTACCCTCGTGCCAGCGAACCAGAAGGGGCGTGCACTGTC
GATCCTGCTCTCCGGCACGACGATCGCAACCGTCGTGGGCGTCCCCGCCGGGGCACT
GCTCGGCACAGCGCTGGGCTGGCGAACGACGTTCTGGGCGATCGCCATCCTCTGTATT
CCCGCGGCCGTTGGAGTCATTCGTGGCGTCACGAACAATGTTGGTCGGAGCGAGACTA
GCGCGACCTCACCAAGGCTCCGTGTCGAGCTCAGCCAGTTGGCGACGCCGCGGCTCA
TCCTGGCCATGGCACTCGGAGCGCTGATCAACGGAGGGACCTTTGCGGCATTCACCTT
CCTGGCACCCATCGTGACCGAGACCGCGGGCTTGGCCGAAGCGTGGGTGTCCGTCGC
GCTGGTGATGTTCGGCATCGGATCGTTCCTTGGCGTCACGATCGCAGGACGACTATCA
GATCAACGACCTGGCCTCGTGCTCGCAGTCGGCGGACCGCTATTGCTGACAGGCTGGA
TCGTGTTGGCAGTGGTCGCATCTCATCCCGTTGCGCTTATCGTCCTCGTCCTCGTTCAG
GGATTCCTGTCGTTCGGCGTCGGCAGTACTCTGATCACGCGTGTGCTGTATGCAGCAT
CGGGTGCGCCAACGATGGGCGGTTCGTACGCAACCGCAGCATTGAATATCGGAGCTGC
AGCGGGGCCCGTGCTTGGTGCGCTCGGGCTCGCGACCGGGCTGGGGCTGCTCGCGC

CGGTTTGGGTCGCTTCGGTGCTGACAGCGATCGCTCTCGTCATCATGCTTCTCACCAGA
CGCGCGCTTACGAAGACCGCGGCGGAGGCCAATTGCATATGCGGTGTGAAATACCGCA
CAGATGCGTAAGGAGAAAATACCGCATCAGGCGCTCTTCCGCTTCCTCGCTCACTGACT
CGCTGCGCTCGGTCGTTCGGCTGCGGCGAGCGGTATCAGCTCACTCAAAGGCGGTAAT
ACGGTTATCCACAGAATCAGGGGATAACGCAGGAAAGAACATGTGAGCAAAAGGCCAG
CAAAAGGCCAGGAACCGTAAAAAGGCCGCGTTGCTGGCGTTTTTCCATAGGCTCCGCC
CCCCTGACGAGCATCACAAAAATCGACGCTCAAGTCAGAGGTGGCGAAACCCGACAGG
ACTATAAAGATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGGCGCTTTCT
CATAGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAGGA
TTAGCAGAGCGAGGTATGTAGGCGGTGCTACAGAGTTCTTGAAGTGGTGGCCTAACTA
CGGCTACACTAGAAGAACAGTATTTGGTATCTGCGCTCTGCTGAAGCCAGTTACCTTCG
GAAAAAGAGTTGGTAGCTCTTGATCCGGCAAACAAACCACCGCTGGTAGCGGTGGTTTT
TTTGTTTGCAAGCAGCAGATTACGCGCAGAAAAAAAGGATCTCAAGAAGATCCTTTGATC
TTTTCTACGGGGTCTGACGCTCAGTGGAACGAAAACTCACGTTAAGGGATTTTGGTCAT
GAGATTATCAAAAAGGATCTTCACCTAGATCCTTTTAAATTAAAAATGAAGTTTTAAATCA
ATCTAAAGTATATATGAGTAAACTTGGTCTGACAGTTACCAATGCTTAATCAGTGAGGCA
CCTATCTCAGCGATCTGTCTATTTCGTTCATCCATAGTTGCCTGACTCCCCGTCGTGTAG
ATAACTACGATACGGGAGGGCTTACCATCTGGCCCCAGTGCTGCAATGATACCGCGAG
ACCCACGCTCACCGGCTCCAGATTTATCAGCAATAAACCAGCCAGCCGGAAGGGCCGA
GCGCAGAAGTGGTCCTGCAACTTTATCCGCCTCCATCCAGTCTATTAATTGTTGCCGGG
AAGCTAGAGTAAGTAGTTCGCCAGTTAATAGTTTGCGCAACGTTGTTGCCATTGCTACA
GGCATCGTGGTGTCACGCTCGTCGTTTGGTATGGCTTCATTCAGCTCCGGTTCCCAACG
ATCAAGGCGAGTTACATGATCCCCCATGTTGTGCAAAAAAGCGGTTAGCTCCTTCGGTC
CTCCGATCGTTGTCAGAAGTAAGTTGGCCGCAGTGTTATCACTCATGGTTATGGCAGCA
CTGCATAATTCTCTTACTGTCATGCCATCCGTAAGATGCTTTTCTGTGACTGGTGAGTAC
TCAACCAAGTCATTCTGAGAATAGTGTATGCGGCGACCGAGTTGCTCTTGCCCGGCGTC
AATACGGGATAATACCGCGCCACATAGCAGAACTTTAAAAGTGCTCATCATTGGAAAAC
GTTCTTCGGGGCGAAAACTCTCAAGGATCTTACCGCTGTTGAGATCCAGTTCGATGTAA
CCCACTCGTGCACCCAACTGATCTTCAGCATCTTTTACTTTCACCAGCGTTTCTGGGTGA
GCAAAAACAGGAAGGCAAAATGCCGCAAAAAAGGGAATAAGGGCGACACGGAAATGTT
GAATACTCATACTCTTCCTTTTTCAATATTATTGAAGCATTTATCAGGGTTATTGTCTCAT
GAGCGGATACATATTTGAATGTATTTAGAAAAATAAACAAATAGGGGTTCCGCGCACATT
TCCCCGAAAAGTGCCACCTGACGTCTAAGAAACCATTATTATCATGACATTAACCTATAA
AAATAGGCGTATCACGAGGCCCTTTCGTCTTCAA

### EXAMPLES

### EXAMPLE 1: METHODS

### Recombinant NGAL protein production in E. coli BL21

LCN2 gene coding for NGAL protein was cloned in pETM14 N-terminal his tagged vector and transformed in E. coli BL21 cells. The recombinant human (rhNGAL) protein was expressed in auto-induction LB media o/n at 25 °C. Cells were lysed by French press and proteins were purified by a HiTrap Ni2+ column followed by desalting and a size exclusion. From 1 L culture, 98 mg of NGAL protein was obtained.

### Toxicity test of human recombinant NGAL produced in E. coli (rhNGAL) on C. acnes culture

C. *acnes* KPA171202 was inoculated to a starting OD600 of 0,05. rhNGAL protein (stock 1,35 mg/mL) produced in *E. coli* BL21 was diluted and added to the cultures at final concentrations of 0, 100, 500 and. 1000 ng/mL. Bacteria was incubated with rhNGAL protein at 37°C degrees, 110 rpm for 24 h. After 24h toxicity to cultures was measured by their OD600.

### MTT viability assay of human recombinant NGAL produced in E. coli (rhNGAL) on human SZ95 sebocytes

Human SZ95 sebocytes were seeded in a 12-well plate to a density of 2×10^5 cells/well and incubated in a humidified atmosphere (37 °C, 5 % CO2) until 80 % confluency in Sebomed media supplemented with 10% heat inactivated FBS, 100 U/mL Pen/Strep,1mM CaCl2 and 5 ng/ml human epidermal growth factor (EGF; Sigma-Aldrich Co.). Then, SZ95 were incubated with purified rhNGAL protein produced in *E. coli* BL21 cells in concentration ranging from 0, 50, 100, 500 or 1000 ng/mL for 24 h and then subsequently incubated with fresh medium and 10% MTT (5 mg/mL in phosphate buffered saline) for 2 h at 37 °C. Afterwards, the medium was removed carefully and 500 µL of dimethyl sulfoxide (DMSO) 99% purity was added to lysate the cells and dissolve the purple insoluble crystals of MTT. The cell lysate was transferred to a new 96-well plate and then the absorbance was read using a Microplate Autoreader at excitation/emission of 540/630 nm (Tecan). Absorbance values were considered directly proportional to cell viability.

Recombinant NGAL incubation with Pci SEB-Cau sebocytes to measure sebum reduction Pci-SEB_Cau human iPSC derived sebocytes (Phenocell) were seeded in a 24 well plate at cell density of 25000 viable cells/cm² in PhenoCULT SEB-medium supplemented with 1/1000 Supplement A. Cells were grown in a humidified incubator (37 °C, 5 % CO2) for 3 days before being exposed and starting the assay. Experiment was performed in triplicates being treated with either vehicle, 5 µM AA (arachidonic acid), 5 µM AA+ 10 µM ISO (13-cis retinoic acid), 5 µM AA+ 50 ng/mL rhNGAL (from *E. coli* BL21), 5 uM AA+ 50 ng/mL pBR13 NGAL (from C. *acnes* KPA171202, see Table 1), 5 µM AA+ 50 ng/mL pBR14 NGAL (C. *acnes* KPA171202, see Table 1), 5 µM AA+ 50 ng/mL pBR16 NGAL (C. *acnes* KPA171202, see Table 1). Cells were exposed for 48 h and then cell lipid content measured by BODIPY dye.

### Transformation of C. acnes:

C. *acnes* competent cells are prepared as previously described (Meike Sorensen, et al. Mutagenesis of Propionibacterium acnes and analysis of two CAMP factor knock-out mutants,Journal of Microbiological Methods,Volume 83, Issue 2,2010,Pages 211-216,ISSN 0167-7012,https://doi.org/10.1016/j.mimet.2010.09.008.). Briefly, C. *acnes* cells are inoculated to a starting OD600 of 0,1 and grown for 24 h anaerobically, 110 rpm at 37°C degrees. Bacterial culture is centrifuged for 10 min, 4200 rpm and washed 7 times in total with ice-cold 272 mM Sucrose buffer. 10 µl of bacterial pellet is diluted in 30 µl of 272 mM Sucrose buffer and mixed with 8 µg methylated plasmid DNA. Methylated plasmid DNA was produced by shuttling the plasmid through a IIIB methylation proficient *E. coli* dam-dcm- strain. This *E. coli* strain was engineered to produce the C. *acnes* IIIB methylase. The bacteria were transformed with the plasmid DNA by electroporation using a Biorad gene pulser apparatus (BioRad) at 1500V, 400 ohm, 25 uF, 1 mm. Cells were recovered for 24 h on a Brucella agar plate anaerobically and afterwards selected on a Brucella plate supplemented with 10 µg/mL erythromycin. Plates were incubated anaerobically for 7 days at 37°C degrees. Positive clones were checked by junction PCR and WGS for positive gene insertion/ plasmid transformation.

### Cloning of different variations of promoter and signalling peptides:

Six different constructs with 2 promoters and 3 secretion peptides were cloned in pGEM-Teasy vector together with 500 bp homology upstream and downstream of gene to be replaced (camp2, camp1, roxP, tdk or others) as previously described in Meike Sorensen, et al. (https://doi.org/10.1016/j.mimet.2010.09.008), Nazipi S, et al. (The Skin Bacterium Propionibacterium acnes Employs Two Variants of Hyaluronate Lyase with Distinct Properties. Microorganisms. 2017 Sep 12;5(3):57. Doi: 10.3390/microorganisms5030057). And Allhorn, M et al. (A novel enzyme with antioxidant capacity produced by the ubiquitous skin colonizer Propionibacterium acnes. Sci Rep 6, 36412 (2016). https://doi.org/10.1038/srep36412) or cloned into pBRESP36A plasmid. pBRESP36A plasmid is a replicative plasmid in C. acnes, and the transformation efficiency with this vector (or derivatives) is much higher than with the suicide vector for homologous recombination, making it more suitable for protein production studies (see Jore JP, et al. Efficient transformation system for Propionibacterium freudenreichii based on a novel vector. Appl Environ Microbiol. 2001;67(2):499-503. doi:10.1128/AEM.67.2.499-503.2001). All gene constructs were fused to a C- terminal histidine tag for purification or western blot analysis.

**Table 1: construct, promoter name and secretion peptide name.**

| Construct | Promoter name | Secretion peptide name |
|---|---|---|
| pBR13 | PPA1340 | PPA1939 |
| pBR14 | PPA1340 | PPA0687 |
| pBR16 | PPA1340 | PPA1340 |
| pBR18 | PPA1939 | PPA1939 |
| pBR19 | PPA1939 | PPA0687 |
| pBR20 | PPA1939 | PPA1340 |

### Recombinant NGAL protein purification of C. acnes supernatant

Proteins of 100 mL filtered supernatant were purified by a HiTrap Ni²⁺ column followed by desalting and a size exclusion. Total proteins were quantified by Qubit Protein assay kit (Invitrogen), concentrations and final protein yields of pBR13 (0,46 mg), pBR14 (0,14 mg), pBR16 (0,50 mg) obtained.

### C. acnes culture for protein production and secretion

Transformed C. *acnes* KPA171202 was inoculated in 50 mL BHI medium supplemented with 10 µg/mL erythromycin to a starting OD600 of 0,1 and grown anaerobically for 48 h until reaching an OD of 1,2. Cells were spun down for 10 min, 5000 rpm. Pellet was resuspended in 1 mL S30 buffer, lysed by a FastPrep FP120 for 25 sec, speed 6.5, 2 cycles and centrifuged full speed, 30 min at 4 degrees. The supernatant was filtered through 0,22 um. Both filtered supernatant and lysate were used for TCA precipitation of secreted proteins.

### Deoxycholate-Trichloroacetic acid (TCA) precipitation of C. acnes produced NGAL protein

Proteins from lysate and 50 mL filtered supernatants were TCA precipitated. In summary, protein containing supernatants were treated for 10 min at RT with deoxycholate (DOC) and then precipitated using 10% trichloroacetic acid (TCA) (Sigma Aldrich) on ice for 30 min. Samples were centrifuged for 10000xg, 4 °C, 15 min and the protein pellet was washed twice in pure ice-cold acetone, air-dried and resuspended in 100 µl of 25 mM Tris-HCI buffer (pH 8,0).

### Western blot

20 µl of TCA precipitated protein was mixed with SDS-PAGE sample loading buffer, boiled for 5 min at 95°C and electrophoresed on a NuPAGE 4-12% Bis-Tris SDS-gel (Invitrogen). Proteins were transferred to a Hybond-P polyvinylidene difluoride membrane using a Invitrogen Mini Transblot apparatus. The membrane was blocked in PBS-0.05% Tween-20 and 5% skimmed milk (blocking solution) for 1 h at room temperature, and incubated with specific antibodies against Histidine tag (BioRad), 1:800 dilution in blocking solution overnight at 4°C, followed by incubation with peroxidase-conjugated anti-mouse IgG antibody (1:10000) for 1 h at room temperature. The antibody-protein complex was visualized using the ECL Plus Western blotting detection system (GeHealthcare).

### Inhibiting thymidine kinase (tdk) in wild type C. acnes for a potential negative selection

To test if tdk could be used as a negative selection in C. acnes, we tested its functionality with the known inhibitor 5-fluoro-2'-deoxyuridine (FUDR). We plated serial dilutions of C. acnes wild type KPA171202 from 10^-1 to 10^-8 in 10 µl drops on Brucella agar plates containing 0 or 50 µg/mL FUDR. Plates were incubated for 7 days at 37 °C anaerobically and checked for potential growth. If know growth was seen, the inhibitor FUDR is functional against the tdk gene.

### EXAMPLE 2: Results

### Viability test of SZ95 human sebocytes and C. acnes treated with rhNGAL

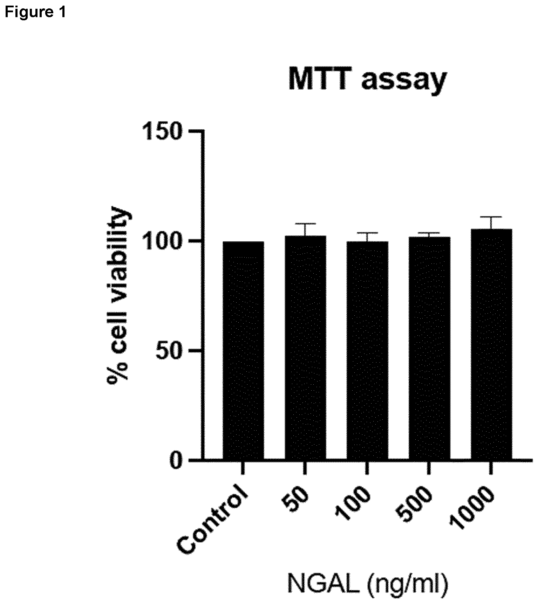

To investigate the effects of rhNGAL protein produced in *E. coli* on the proliferation of human sebocytes, we used the human SZ95 sebocyte cell line. In the present study, the antiproliferative effect of NGAL on sebocytes was determined. Therefore, cells were treated with various concentrations of NGAL and cell proliferation and viability assessed by a MTT (colorimetric assay for assessing cell metabolic activity) assay. No significant effect was found after 24 h on the viability and proliferation of the SZ95 cells in a dose dependent manner (Figure 1).

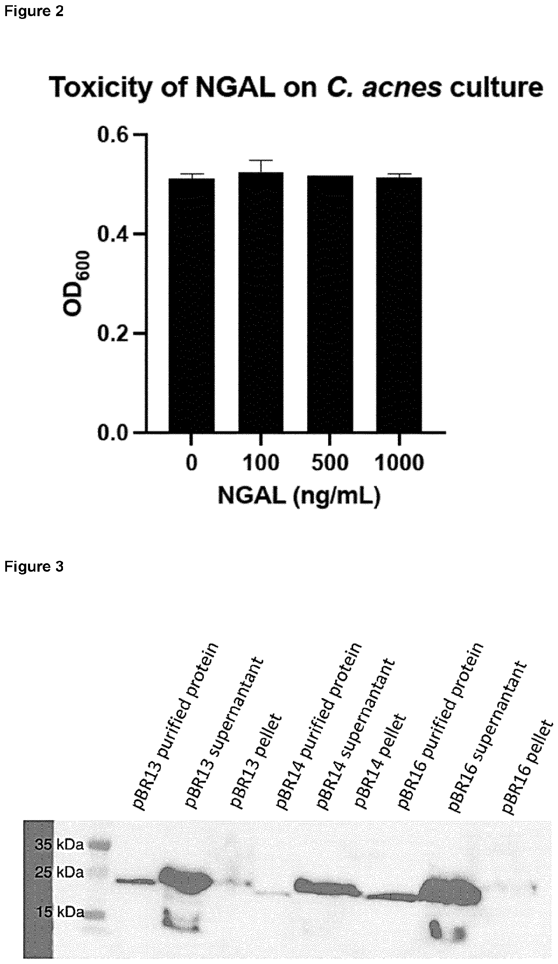

As another test, we assessed rhNGAL toxicity on C. *acnes* culture using several doses. OD600 was measured after 24 h incubation and no toxic effect observed on C. *acnes* bacteria measured on its growth comparing to the un-treated control (Figure 2). The used rhNGAL protein was expressed and purified from *E. coli* BL21 cells (see Example 1 above).

### Protein production and secretion of NGAL in C. acnes

Total protein production of NGAL protein was measured of the three different constructs pBR13, pBR14 and pBR16 (see Table 1) in the secreted fraction vs. cytosolic fraction of the TCA precipitated protein. Secreted and cytosolic fractions were visualized by Western blot and compared to 40 µg (pBR13), 10 µg (pBR14) and 25 µg (pBR16) of purified protein. Western blot showed higher amount of protein in the secreted fraction (Figure 3).

### rhNGAL reduces sebum production in PCi-SEB Cau sebocytes in vitro

To test the ability of purified rhNGAL produced in *E. coli* or C. *acnes* in reducing sebum in PCi-SEB Cau we induced sebum production with 5 µM Arachidonic acid (AA) and treated them simultaneously with rhNGAL and isotretinoin for 48h. It could be seen, that all NGAL proteins, i.e., rhNGAL produced in *E. coli* (1,7-fold), pBR13 (2,5-fold), pBR14 (1,5-fold), pBR16 (1,6-fold) and isotretinoin (1,8-fold) induced a significant decrease in sebum production compared to AA treated cells (Figure 4). Interestingly, the NGAL protein produced in C. *acnes* (pBR13) significantly reduced sebum 1,3-fold in comparison to the positive control isotretinoin (Figure 4).

### TDK test

We checked the ability of growth of C. *acnes* KPA171202 wild type strain on Brucella agar plates, supplemented with or without 50 µg/mL FUDR (5-fluoro-2'-deoxyuridine). The results (Fig. 5), show that bacteria could only grow in absence of the thymidilate synthase inhibitor (FUDR), showing that that the inhibitor is functional against the thymidine kinase. These results shown that a thymidine kinase (tdk) knock out could be used to select for our LCN2 insertion by using the inhibitor and therefore not require an antibiotic resistance. The absence of antibiotic resistance is important for the application of the bacteria on the human skin.

### Discussion

In this study, we assessed the effect of bacterial NGAL on human sebocytes. The protein to be tested was produced in *E. coli* BL21 or C. *acnes* KPA171202. Three different plasmid versions of NGAL with combinations of regulatory parts (pBR13, pBR14 and pBR16) were transformed in C. *acnes* and tested for the ability in producing and secreting NGAL protein. We verified the presence of NGAL protein in C. *acnes* supernatant by protein purification and western blot and showed that pBR13, pBR14 and pBR16 were able to secrete NGAL, whereas an increased production was observed in pBR13 and pBR16.

Additionally, NGAL protein had no effect on the viability of C. *acnes* and on human SZ95 sebocytes in vitro, which is contrary to what is has been previously shown (Klausen et al. 2005.

On mouse and man: neutrophil gelatinase associated lipocalin is not involved in apoptosis or acute response. Eur J Haematol: 75: 332-340 and Kimberly Ruth Lumsden. 2011. The Innate Immune Protein Neutrophil Gelatinase- Associated Lipocalin Is Involved in The Early Therapeutic Response To 13-Cis Retinoic Acid In Acne Patients. PhD Dissertation).

We also measured ability of bacterial NGAL in reducing sebum from stimulated PCi SEB-Cau sebocytes in vitro. A significant reduction in sebum was observed after 48h which was comparable to isotretinoin treatment. The C. *acnes* produced NGAL (pBR13) showed a 1,3-fold decrease in sebum compared to isotretinoin treatment.

In this study we demonstrate that C. *acnes* can produce therapeutic amounts of NGAL and is able to secrete the protein to the supernatant. Filtered supernatant of C. *acnes* was able to reduce sebum comparably to isotretinoin in an in vitro sebocytes model. This data suggests the beneficial use of NGAL produced in bacteria *(E. coli* and C. *acnes),* together with the potential therapeutic use of NGAL producing C. *acnes* as a shuttle for cosmetic purposes or to reduce sebum and treat *acne vulgaris*

## Claims

1. A human neutrophil gelatinase-associated lipocalin (NGAL) protein derived from a culture of a recombinant bacterium, wherein said NGAL protein is obtained or obtainable from a method comprising the steps of:
a) culturing in a suitable media a recombinant bacterium **characterized in that** it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b),
**for use** in inhibiting sebum production in a human epithelial cell, preferably in a sebocyte.

2. A human neutrophil gelatinase-associated lipocalin (NGAL) protein derived from a culture of a recombinant bacterium, wherein said NGAL protein is obtained or obtainable from a method comprising the steps of:
a) culturing in a suitable media a recombinant bacterium **characterized in that** it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b),
**for use** in the treatment of *acne vulgaris,* urticaria, eczema, rosacea and/or psoriasis.

3. **A non-therapeutical cosmetic use** of a human neutrophil gelatinase-associated lipocalin (NGAL) protein derived from a culture of a recombinant bacterium, wherein said NGAL protein is obtained or obtainable from a method comprising the steps of:
a) culturing in a suitable media a recombinant bacterium **characterized in that** it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b),
preferably to prevent, reduce and/or ameliorate skin aging.

4. The use according to any of claims 1 or 2, or the method according to claim 3, wherein the recombinant bacterium is *Cutibacterium acnes* or *Escherichia coli.*

5. The use according to any of claims 1 or 2, or the method according to claim 3, wherein the human neutrophil gelatinase-associated lipocalin (NGAL) protein has at least 85% amino acid sequence identity over the full length to SEQ ID NO: 1.

6. The use according to any of claims 1 or 2, or the method according to claim 3, wherein the recombinant bacterium is *Cutibacterium acnes* and wherein the human neutrophil gelatinase-associated lipocalin (NGAL) protein has at least 85% amino acid sequence identity over the full length to SEQ ID NO: 1.

7. A **method** for producing the active ingredient of a pharmaceutical composition, wherein said active ingredient is a human neutrophil gelatinase-associated lipocalin (NGAL) protein that is capable of inhibiting sebum production in a human epithelial cell, preferably in a sebocyte, wherein the method comprises the steps of:
a) culturing in a suitable media a recombinant bacterium **characterized in that** it expresses a human neutrophil gelatinase-associated lipocalin (NGAL) protein, preferably a secretory NGAL protein,
b) collecting the NGAL protein from the culture of a), preferably from the supernatant of the culture of a),
c) optionally, isolating the NGAL collected in b).

8. The method according to claim 7, wherein the recombinant bacterium is *Cutibacterium acnes* or *Escherichia coli.*

9. The method according to claims 7 or 8, wherein the human neutrophil gelatinase-associated lipocalin (NGAL) protein has at least 85% amino acid sequence identity over the full length to SEQ ID NO: 1.

10. The method according to any of claims 7 to 9, wherein the recombinant bacterium is *Cutibacterium acnes* and wherein the human neutrophil gelatinase-associated lipocalin (NGAL) protein has at least 85% amino acid sequence identity over the full length to SEQ ID NO: 1.

11. A **recombinant** *Cutibacterium acnes* **characterized in that** it expresses the human neutrophil gelatinase-associated lipocalin (NGAL) protein.

12. The recombinant *Cutibacterium acnes* according to claim 11, wherein the human neutrophil gelatinase-associated lipocalin (NGAL) protein has at least 85% amino acid sequence identity over the full length to SEQ ID NO: 1.

13. The recombinant *Cutibacterium acnes* according to any of the claims 11 or 12, wherein the nucleic acid encoding for the human neutrophil gelatinase-associated lipocalin (NGAL) is operably linked to a promoter selected from the list consisting of camp2 promoter, camp1 promoter or roxP promoter.
